# EUROPEAN PATENT SPECIFICATION

(11) **EP 3 452 487 B1**
(45) Date of publication and mention of the grant of the patent: **08.09.2021**
(21) Application number: 17723483.8
(22) Date of filing: 05.05.2017
(51) Int. Cl.: C07F 5/06

(54) **CATALYTIC SUPPORT AND USES THEREOF**
KATALYSATORTRÄGER UND VERWENDUNGEN DAVON
SUPPORT CATALYTIQUE ET LEURS UTILISATIONS

(30) Priority: 06.05.2016 GB 201607989
(43) Date of publication of application: 13.03.2019
(73) Proprietor: SCG Chemicals Co., Ltd., Bangkok 10800 (TH)
(72) Inventor: O'HARE, Dermot, Oxford OX1 3TA (GB); BUFFET, Jean-Charles, Oxford OX1 3TA (GB); KILPATRICK, Alexander, Oxford OX1 3TA (GB); SOMSOOK, Ekasith, Ratchathewi Bangkok 10400 (TH); NEALMONGKOLRATTANA, Nitiphat, Bangsue Bangkok 10800 (TH); CHAROENCHAIDET, Sumate, Bangsue Bangkok 10800 (TH); SRIPOTHONGNAK, Saovalak, Bangsue Bangkok 10800 (TH)
(74) Representative: HGF
(86) International application number: PCT/GB2017/051257
(87) International publication number: WO 2017/191467

(56) References cited:
- WO-A2-03/033545
- US-B1- 6 664 208
- US-B1- 6 696 379

## Description

### INTRODUCTION

The present invention relates to catalytic support materials and uses thereof. More particularly, the present invention relates to catalytic support materials based on solid polymethylaluminoxane, as well as the use of such materials in polymerisation of olefins.

### BACKGROUND OF THE INVENTION

It is well known that ethylene (and α-olefins in general) can be readily polymerised at low or medium pressures in the presence of certain transition metal catalysts. These catalysts are generally known as Zeigler-Natta type catalysts.

A particular group of these Ziegler-Natta type catalysts, which catalyse the polymerization of ethylene (and α-olefins in general), comprise an aluminoxane activator and a metallocene transition metal catalyst. Metallocenes comprise a metal bound between two η⁵-cyclopentadienyl type ligands. Generally the η⁵-cyclopentadienyl type ligands are selected from η⁵-cyclopentadienyl, η⁵-indenyl and η⁵-fluorenyl.

Catalytic reactions involving metallocene-based Ziegler-Natta catalysts have traditionally employed the catalyst in solution phase. However, this technique has a number of drawbacks, most notably the difficulty of effectively separating the catalyst from the reaction medium and then recycling it for further use.

US6696379B1 describes compounds that are useful as catalyst activator components, in particular those adapted for use in the polymerization of unsaturated compounds.

Given the high value that industry places on polyethylene (as well as other polyolefins), there is a need for improved solid-phase support materials capable of effectively supporting metallocene-based Ziegler-Natta catalysts.

The present invention was devised with the foregoing in mind.

### SUMMARY OF THE INVENTION

According to a first aspect of the present invention there is provided a solid-phase support material suitable for supporting a metallocene catalytic compound, the solid-phase support material comprising a solid polymethylaluminoxane modified by reaction with a compound of formula (I) or (II) defined herein, wherein the mole ratio of the compound of formula (I) or (II) to the solid polymethylaluminoxane within the solid-phase support material ranges from 0.0001:1 to 0.3:1.

According to a further aspect of the present invention, there is provided a method of preparing a solid-phase support material as claimed in any preceding claim, the method comprising the steps of:
a) providing a solid polymethylaluminoxane in a first solvent;
b) contacting the solid polymethylaluminoxane of step a) with one or more compounds of formula (I) or (II) as defined herein; and
c) isolating the product formed from step b);
wherein the mole ratio of the compound of formula (I) or (II) to the solid polymethylaluminoxane used in step b) ranges from 0.0001:1 to 0.3:1.

According to a further aspect of the present invention, there is provided a solid-phase support material obtained, directly obtained or obtainable by a process defined herein.

According to a further aspect of the present invention, there is provided a use of a solid-phase support material defined herein as a solid-phase support for supporting a metallocene catalytic compound.

According to a further aspect of the present invention, there is provided a catalytic composition comprising:
a) a compound of formula (III) defined herein; and
b) a solid-phase support material as defined herein.

According to a further aspect of the present invention, there is provided a method for the preparation of a catalytic composition defined herein, the method comprising the steps of:
a) providing a solid-phase support material as defined herein in a suitable solvent
b) contacting the solid-phase support material of step a) with a compound of formula (III) defined herein; and
c) isolating the product resulting from step b).

According to a further aspect of the present invention, there is provided a catalytic composition obtained, directly obtained or obtainable by a process defined herein.

According to a further aspect of the present invention, there is provided a use of a catalytic composition as defined herein as a polymerisation catalyst for the preparation of a polyolefin.

According to a further aspect of the present invention, there is provided a process for forming a polyolefin (e.g. a polyethylene), the process comprising the step of reacting olefin monomers in the presence of a catalytic composition defined herein.

### DETAILED DESCRIPTION OF THE INVENTION

### Definitions

The term "alkyl" as used herein includes reference to a straight or branched chain alkyl moieties, typically having 1, 2, 3, 4, 5 or 6 carbon atoms. This term includes reference to groups such as methyl, ethyl, propyl (n-propyl or isopropyl), butyl (n-butyl, sec-butyl or tert-butyl), pentyl (including neopentyl), hexyl and the like. In particular, an alkyl may have 1, 2, 3 or 4 carbon atoms.

The term "alkenyl" as used herein include reference to straight or branched chain alkenyl moieties, typically having 2, 3, 4, 5 or 6 carbon atoms. The term includes reference to alkenyl moieties containing 1, 2 or 3 carbon-carbon double bonds (C=C). This term includes reference to groups such as ethenyl (vinyl), propenyl (allyl), butenyl, pentenyl and hexenyl, as well as both the *cis* and *trans* isomers thereof.

The term "alkynyl" as used herein include reference to straight or branched chain alkynyl moieties, typically having 2, 3, 4, 5 or 6 carbon atoms. The term includes reference to alkynyl moieties containing 1, 2 or 3 carbon-carbon triple bonds (C≡C). This term includes reference to groups such as ethynyl, propynyl, butynyl, pentynyl and hexynyl.

The term "alkoxy" as used herein include reference to -O-alkyl, wherein alkyl is straight or branched chain and comprises 1, 2, 3, 4, 5 or 6 carbon atoms. In one class of embodiments, alkoxy has 1, 2, 3 or 4 carbon atoms. This term includes reference to groups such as methoxy, ethoxy, propoxy, isopropoxy, butoxy, tert-butoxy, pentoxy, hexoxy and the like.

The term "aryl" as used herein includes reference to an aromatic ring system comprising 6, 7, 8, 9 or 10 ring carbon atoms. Aryl is often phenyl but may be a polycyclic ring system, having two or more rings, at least one of which is aromatic. This term includes reference to groups such as phenyl, naphthyl and the like.

The term "carbocyclyl" as used herein includes reference to an alicyclic moiety having 3, 4, 5, 6, 7 or 8 carbon atoms. The group may be a bridged or polycyclic ring system. More often cycloalkyl groups are monocyclic. This term includes reference to groups such as cyclopropyl, cyclobutyl, cyclopentyl, cyclohexyl, norbornyl, bicyclo[2.2.2]octyl and the like.

The term "heterocyclyl" as used herein includes reference to a saturated (e.g. heterocycloalkyl) or unsaturated (e.g. heteroaryl) heterocyclic ring moiety having from 3, 4, 5, 6, 7, 8, 9 or 10 ring atoms, at least one of which is selected from nitrogen, oxygen, phosphorus, silicon and sulphur. In particular, heterocyclyl includes a 3- to 10-membered ring or ring system and more particularly a 5- or 6-membered ring, which may be saturated or unsaturated.

A heterocyclic moiety is, for example, selected from oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2*H*-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, isoindolyl, 3*H*-indolyl, indolyl, benzimidazolyl, cumaryl, indazolyl, triazolyl, tetrazolyl, purinyl, 4*H*-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, β-carbolinyl, phenanthridinyl, acridinyl, perimidinyl, phenanthrolinyl, furazanyl, phenazinyl, phenothiazinyl, phenoxazinyl, chromenyl, isochromanyl, chromanyl and the like.

The term "heteroaryl" as used herein includes reference to an aromatic heterocyclic ring system having 5, 6, 7, 8, 9 or 10 ring atoms, at least one of which is selected from nitrogen, oxygen and sulphur. The group may be a polycyclic ring system, having two or more rings, at least one of which is aromatic, but is more often monocyclic. This term includes reference to groups such as pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, triazinyl, phthalazinyl, 2H-chromenyl, oxazolyl, isoxazolyl, thiazolyl, isoindolyl, indazolyl, purinyl, isoquinolinyl, quinazolinyl, pteridinyl and the like.

The term "halogen" or "halo" as used herein includes reference to F, Cl, Br or I. In a particular, halogen may be F or Cl, of which Cl is more common.

The term "substituted" as used herein in reference to a moiety means that one or more, especially up to 5, more especially 1, 2 or 3, of the hydrogen atoms in said moiety are replaced independently of each other by the corresponding number of the described substituents. The term "optionally substituted" as used herein means substituted or unsubstituted.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

### Solid phase support material

As discussed hereinbefore, the present invention provides a solid-phase support material suitable for supporting a metallocene catalytic compound, the solid-phase support material comprising a solid polymethylaluminoxane modified by reaction with a compound of formula (I) or (II) shown below: 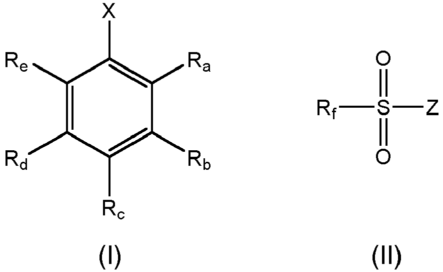 wherein
X is hydroxyl or a group B(Y)₂ or a group Al(Y)₂,
   wherein each Y is independently selected from hydroxyl, phenyl and naphthalenyl, any of which may be optionally substituted with one or more groups selected from halo, hydroxyl, (1-4C)alkyl, (2-4C)alkenyl, (2-4C)alkynyl and trihaloalkyl;
Rₐ and R_{b} are independently hydrogen, hydroxyl, (1-4C)alkyl, (2-4C)alkenyl, (2-4C)alkynyl, halo or trihaloalkyl, or
Rₐ and R_{b} are linked, such that, when taken with the atoms to which they are attached, they form a 6-membered aromatic ring that is optionally substituted by one or more groups selected from halo, (1-4C)alkyl, (2-4C)alkenyl, (2-4C)alkynyl and trihaloalkyl;
R_{c}-Rₑ are independently hydrogen, hydroxyl, (1-4C)alkyl, (2-4C)alkenyl, (2-4C)alkynyl, halo or trihaloalkyl;
Z is hydroxyl or a group -NRₓR_{y},
   wherein Rₓ and R_{y} are independently selected from hydrogen and (1-4C)alkyl;
   R_{f} is (1-4C)alkyl, (2-4C)alkenyl, (2-4C)alkynyl, trihaloalkyl or a phenyl group that is optionally substituted with one or more groups selected from hydroxyl, (1-4C)alkyl, (2-4C)alkenyl, (2-4C)alkynyl, halo and trihaloalkyl;
wherein the mole ratio of the compound of formula (I) or (II) to the solid polyaluminoxane within the solid-phase support material ranges from 0.0001:1 to 0.3:1.

The solid-phase support materials of the invention present a number of advantages over other solid-phase support material. Perhaps most notably, when used in the preparation of supported metallocene-based Ziegla-Natta catalysts for use in the polymerisation of olefins, the solid-phase support materials of the invention give rise to a marked increase in catalytic activity.

It will be understood that the term "solid polymethylaluminoxane" and "solid MAO" as used herein synonymously refer to a solid-phase material having the general formula -[(Me)AlO]ₙ-, wherein n is an integer from 10 to 50. Any suitable solid polymethylaluminoxane may be used..

There exist numerous substantial structural and behavioural differences between solid polymethylaluminoxane and other (non-solid) methyl aluminoxanes. Perhaps most notably, solid polymethylaluminoxane is distinguished from other methyl aluminoxanes (MAOs) as it is insoluble in hydrocarbon solvents and so acts as a heterogeneous support system. The solid polymethylaluminoxanes useful in the preparation of the solid-phase support material of the invention are insoluble in toluene and hexane.

In contrast to non-solid (hydrocarbon-soluble) methyl aluminoxanes, which are traditionally used as an activator species in slurry polymerisation or to modify the surface of a separate solid support material (e.g. SiO₂), the solid polyaluminoxanes useful as part of the present invention are themselves suitable for use as solid-phase support materials, without the need for an additional activator. Hence, the solid-phase support materials of the invention are devoid of any other species that could be considered a solid support (e.g. inorganic material such as SiO₂, Al₂O₃ and ZrO₂). Similarly, the only inorganic solid support employed in the catalytic compositions of the invention is the solid-phase support material of the invention (i.e. no additional solid support such as SiO₂, Al₂O₃ and ZrO₂ are necessary). Moreover, given the dual function of the solid-phase support materials of the invention (as catalytic support and activator species), the catalytic compositions of the invention contain no additional catalytic activator species.

In an embodiment, the solid polymethylaluminoxane is prepared by heating a solution containing polymethylaluminoxane and a hydrocarbon solvent (e.g. toluene), so as to precipitate solid polymethylaluminoxane. The solution containing polymethylaluminoxane and a hydrocarbon solvent may be prepared by reacting trimethyl aluminium and benzoic acid in a hydrocarbon solvent (e.g. toluene), and then heating the resulting mixture.

In an embodiment, the solid polymethylaluminoxane is prepared according to the following protocol:

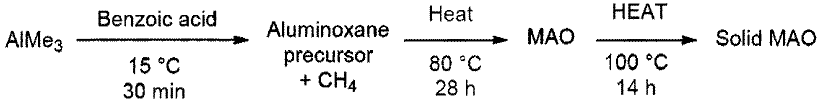

The properties of the solid polymethylaluminoxane can be adjusted by altering one or more of the processing variables used during its synthesis. For example, in the above-outlined protocol, the properties of the solid polymethylaluminoxane may be adjusted by varying the Al:O ratio, by fixing the amount of AlMe₃ and varying the amount of benzoic acid. Exemplary Al:O ratios are 1:1, 1.1:1, 1.2:1, 1.3:1, 1.4:1 and 1.6:1. Suitably the Al:O ratio is 1.2:1 or 1.3:1. Alternatively, the properties of the solid polymethylaluminoxane may be adjusted by fixing the amount of benzoic acid and varying the amount of AlMe₃.

In another embodiment, the solid polymethylaluminoxane is prepared according to the following protocol:

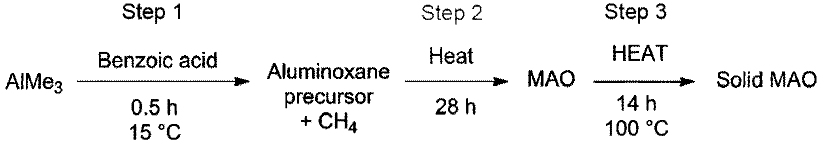

In the above protocol, steps 1 and 2 may be kept constant, with step 2 being varied. The temperature of step 2 may be 70-100°C (e.g. 70°C, 80°C, 90°C or 100°C). The duration of step 2 may be from 12 to 28 hours (e.g. 12, 20 or 28 hours). The duration of step 2 may be from 5 minutes to 24 hours. Step 3 may be conducted in a solvent such as toluene.

In an embodiment, the aluminium content of the solid polymethylaluminoxane falls within the range of 36-41 wt%.

The solid polymethylaluminoxane useful as part of the present invention is characterised by extremely low solubility in toluene and n-hexane. In an embodiment, the solubility in n-hexane at 25°C of the solid polymethylaluminoxane is 0-2 mol%. Suitably, the solubility in n-hexane at 25°C of the solid polymethylaluminoxane is 0-1 mol%. More suitably, the solubility in n-hexane at 25°C of the solid polymethylaluminoxane is 0-0.2 mol%. Alternatively or additionally, the solubility in toluene at 25°C of the solid polymethylaluminoxane is 0-2 mol%. Suitably, the solubility in toluene at 25°C of the solid polymethylaluminoxane is 0-1 mol%. More suitably, the solubility in toluene at 25°C of the solid polymethylaluminoxane is 0-0.5 mol%. The solubility in solvents can be measured by the method described in JP-B(KOKOKU)-H07 42301.

In a particularly suitable embodiment, the solid polymethylaluminoxane is as described in US2013/0059990, WO2010/055652 or WO2013/146337, and is obtainable from Tosoh Finechem Corporation, Japan.

In an embodiment, the mole ratio of the compound of formula (I) or (II) to the solid polymethylaluminoxane within the solid-phase support material ranges from 0.0001:1 to 0.2:1. Suitably, the mole ratio of the compound of formula (I) or (II) to the solid polymethylaluminoxane within the solid-phase support material ranges from 0.0001:1 to 0.1:1. More suitably, the mole ratio of the compound of formula (I) or (II) to the solid polymethylaluminoxane within the solid-phase support material ranges from 0.0001:1 to 0.07:1. Most suitably, the mole ratio of the compound of formula (I) or (II) to the solid polymethylaluminoxane within the solid-phase support material ranges from 0.0001:1 to 0.05:1.

In an embodiment, Z is hydroxyl.

In another embodiment, X is hydroxyl or a group B(Y)₂.

In another embodiment, X is hydroxyl or a group Al(Y)₂.

In another embodiment, each Y is independently selected from hydroxyl, phenyl and naphthalenyl, any of which may be optionally substituted with one or more groups selected from fluoro, hydroxyl, (1-4C)alkyl, (2-4C)alkenyl, (2-4C)alkynyl and trifluoroalkyl.

In another embodiment, each Y is independently selected from hydroxyl or a phenyl group that is optionally substituted with one or more groups selected from fluoro, hydroxyl, (1-4C)alkyl, (2-4C)alkenyl, (2-4C)alkynyl and trifluoroalkyl.

Suitably, each Y is independently selected from hydroxyl or a phenyl group that is optionally substituted with one or more groups selected from fluoro, (1-4C)alkyl and trifluoromethyl.

More suitably, each Y is independently selected from hydroxyl or a phenyl group that is optionally substituted with one or more groups selected from fluoro and trifluoromethyl.

In an embodiment, Rₐ-Rₑ are each independently hydrogen, hydroxyl, (1-4C)alkyl, (2-4C)alkenyl, (2-4C)alkynyl, halo or trihaloalkyl, or
Rₐ and R_{b} are linked, such that, when taken with the atoms to which they are attached, they form a 6-membered aromatic ring that is optionally substituted by one or more groups selected from halo and trihaloalkyl.

Suitably, Rₐ-Rₑ are each independently hydrogen, hydroxyl, (1-4C)alkyl, (2-4C)alkenyl, (2-4C)alkynyl, halo or trihaloalkyl, or
Rₐ and R_{b} are linked, such that, when taken with the atoms to which they are attached, they form a 6-membered aromatic ring that is optionally substituted by four groups, each being independently selected from halo (e.g. fluoro) and trihaloalkyl (e.g. trifluoromethyl).

More suitably, Rₐ-Rₑ are independently hydrogen, hydroxyl, (1-4C)alkyl, 2-4C)alkenyl, (2-4C)alkynyl, fluoro or trifluoroalkyl.

Even more suitably, Rₐ-Rₑ are independently hydrogen, (1-4C)alkyl, fluoro or trifluoroalkyl.

Most suitably, Rₐ-Rₑ are independently hydrogen, methyl, ethyl, tert-butyl, fluoro or trifluoromethyl.

In another embodiment, R_{f} is (1-4C)alkyl, trihaloalkyl or a phenyl group that is optionally substituted with one or more groups selected from hydroxyl, (1-4C)alkyl, halo and trihaloalkyl.

Suitably, R_{f} is (1-4C)alkyl, trihaloalkyl or p-toluenyl.

More suitably, wherein R_{f} is methyl, ethyl, trifluoromethyl or p-toluenyl.

In an embodiment, the compound of formula (I) or formula (II) is selected from one or more of the following:

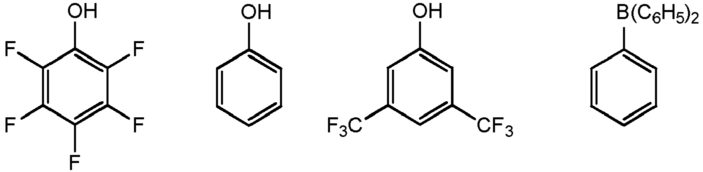

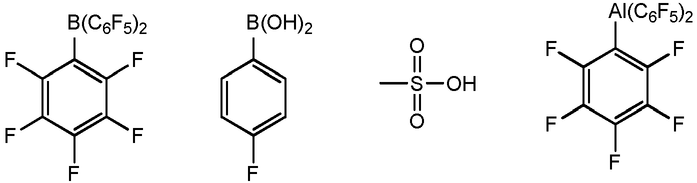

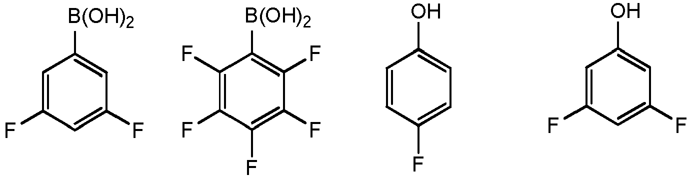

In an embodiment, the compound of formula (I) or formula (II) is selected from one or more of the following:

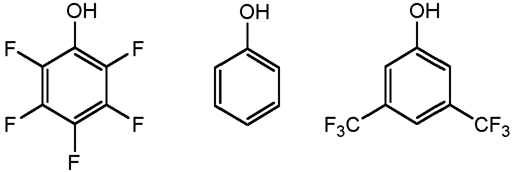

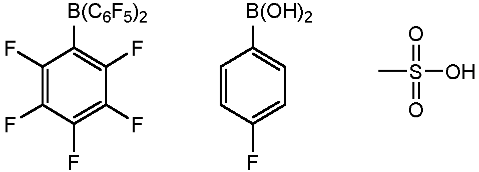

Suitably, the compound of formula (I) or (II) is selected from one or more of the following:

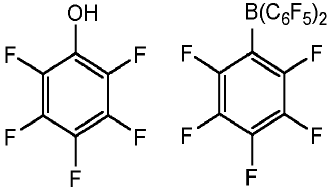

Most suitably, the compound of formula (I) or (II) is:

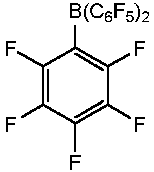

In an embodiment, the solid-phase support material has an aluminium content of 23 to 40 wt%. Suitably, the solid-phase support material has an aluminium content of 28 to 40 wt%. The aluminium content of the solid-phase support material can be determined, for example, by ICP-MS.

In an embodiment, the BET surface area of the solid-phase support material is 10.0 to 20.0 m²mmol_{Al}⁻¹.

In another aspect, the present invention also provides a solid-phase support material suitable for supporting a metallocene catalytic compound, the solid-phase support material comprising a solid polymethylaluminoxane modified by reaction with a compound of formula (I-X) shown below: 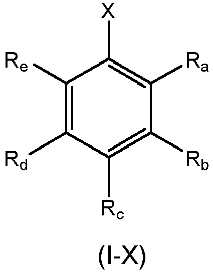 wherein
X is hydroxyl or a group B(Y)₂,
   wherein each Y is pentafluorophenyl; and
Rₐ-Rₑ are each fluoro.

In an embodiment, the mole ratio of the compound of formula (I-X) to the solid polymethylaluminoxane within the solid-phase support material ranges from 0.0001:1 to 1:1.

### Preparation of solid-phase support material

As described hereinbefore, the present invention also provides a method of preparing a solid-phase support material described herein, the method comprising the steps of:
a) providing a solid polymethylaluminoxane in a first solvent;
b) contacting the solid polymethylaluminoxane of step a) with one or more compounds of formula (I) or (II) as defined herein; and
c) isolating the product formed from step b);
wherein the mole ratio of the compound of formula (I) or (II) to the solid polymethylaluminoxane used in step b) ranges from 0.0001:1 to 0.3:1.

The solid-phase support materials of the invention are straightforwardly prepared using mild reaction conditions.

Step a) may comprise the steps of:
i. precipitating a solid polymethylaluminoxane from a reaction medium,
ii. isolating the precipitated solid polymethylaluminoxane from the reaction medium, and
iii. dispersing the isolated solid polymethylaluminoxane in the first solvent.

In an embodiment, the one or more compounds of formula (I) or (II) used in step b) is provided in a second solvent.

The first and second solvents may the same or different. Any suitable aromatic or aliphatic may be used, including toluene, benzene and hexane. In an embodiment, both the first and second solvent are toluene.

Step b) may be conducted at a temperature of 18-150°C. Suitably, step b) is conducted at a temperature of 18-50°C. More suitably, step b) is conducted at a temperature of 20-30°C. Suitably, step b) involves mixing the solid polymethylaluminoxane of step a) with the one or more compounds of formula (I) or (II) as defined herein.

In an embodiment, step b) further comprises the step of sonicating the mixture of the solid polymethylaluminoxane and the one or more compounds of formula (I) or (II). Sonication may be performed for a period of 0.1-24 hours or a period of 0.1-5 hours. Suitably, sonication is performed for a period of 0.5-1.5 hours. When sonication is used, step b) is suitably conducted at a temperature of 10-65°C, preferably 18-50°C. More suitably, when sonication is used, step b) is suitably conducted at a temperature of 18-35°C.

In an embodiment, step b) further comprises the step of sonicating the mixture of the solid polymethylaluminoxane and the one or more compounds of formula (I) or (II), wherein the temperature of the mixture when sonication begins is 15-25°C, and wherein the temperature of the mixture rises to 40-80°C (e.g. 40-65°C) during the course of sonication.

In an embodiment, when step b) comprises sonicating the mixture of the solid polymethylaluminoxane and the one or more compounds of formula (I) or (II), the temperature of the mixture may not rise above 85°C over the course of step b). Suitably, when step b) comprises sonicating the mixture of the solid polymethylaluminoxane and the one or more compounds of formula (I) or (II), the temperature of the mixture may not rise above 65°C over the course of step b).

Any suitable sonicating technique may be used. Suitably, when sonication is used in step b), it is conducted in a sonicating water bath. Suitably, the ultrasonic frequency used in step b) is >15 kHz.

The inventors have surprisingly found that sonicating the mixture of the solid polymethylaluminoxane and the one or more compounds of formula (I) or (II) advantageously obviates the need for conducting step b) at high temperatures, which is believed to result in degradation of the solid-phase support material.

In an embodiment, the mole ratio of the compound of formula (I) or (II) to the solid polymethylaluminoxane used in step b) ranges from 0.0001:1 to 0.2:1. Suitably, the mole ratio of the compound of formula (I) or (II) to the solid polymethylaluminoxane used in step b) ranges from 0.0001:1 to 0.1:1. More suitably, the mole ratio of the compound of formula (I) or (II) to the solid polymethylaluminoxane used in step b) ranges from 0.0001:1 to 0.07:1. Most suitably, the mole ratio of the compound of formula (I) or (II) to the solid polymethylaluminoxane used in step b) ranges from 0.0001:1 to 0.05:1.

In another aspect, the present invention also provides a method of preparing a solid-phase support material described herein, the method comprising the steps of:
a) providing a solid polymethylaluminoxane in a first solvent;
b) contacting the solid polymethylaluminoxane of step a) with one or more compounds of formula (I-X); and
c) isolating the product formed from step b).

### Catalytic compositions

As described hereinbefore, the present invention also provides a catalytic composition comprising:
a) a compound of formula (III) shown below; and
b) a solid-phase support material as defined herein 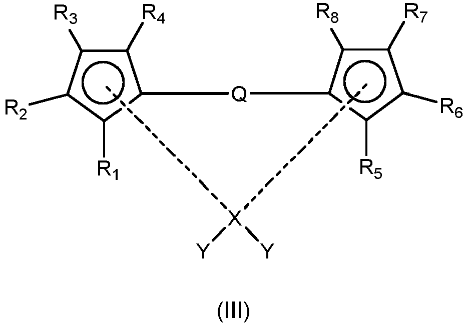 wherein
   R₁ and R₂ are each independently hydrogen or (1-2C)alkyl;
   R₃ and R₄ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl, (1-6C)alkoxy, aryl, heteroaryl, carbocyclic and heterocyclic, wherein each aryl, heteroaryl, carbocyclic and heterocyclic group is optionally substituted with one or more groups selected from (1-6C)alkyl, (2-6C)alkenyl, (2-6C)alkynyl, (1-6C)alkoxy, halo, amino, nitro, cyano, (1-6C)alkylamino, [(1-6C)alkyl]₂amino and -S(O)₂(1-6C)alkyl;
   R₅ and R₆ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl, (1-6C)alkoxy, aryl, heteroaryl, carbocyclic and heterocyclic, wherein each aryl, heteroaryl, carbocyclic and heterocyclic group is optionally substituted with one or more groups selected from (1-6C)alkyl, (2-6C)alkenyl, (2-6C)alkynyl, (1-6C)alkoxy, halo, amino, nitro, cyano, (1-6C)alkylamino, [(1-6C)alkyl]₂amino and -S(O)₂(1-6C)alkyl;
   R₇ and R₈ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl, (1-6C)alkoxy, aryl, heteroaryl, carbocyclic and heterocyclic, wherein each aryl, heteroaryl, carbocyclic and heterocyclic group is optionally substituted with one or more groups selected from (1-6C)alkyl, (2-6C)alkenyl, (2-6C)alkynyl, (1-6C)alkoxy, halo, amino, nitro, cyano, (1-6C)alkylamino, [(1-6C)alkyl]₂amino and -S(O)₂(1-6C)alkyl;
   Q is absent (in which case each cycopentadienyl ring is bound to hydrogen at this position), or is a bridging group selected from -CH₂- or -CH₂CH₂-, either or which may be optionally substituted with one or more groups selected from (1-4C)alkyl, (2-4C)alkenyl, (2-4C)alkynyl and aryl, or Q is a bridging group -Si(R₉)(R₁₀)-,
      wherein Rg and R₁₀ are independently (1-4C)alkyl, (2-4C)alkenyl, (2-4C)alkynyl or aryl;
   X is zirconium or hafnium; and
   each Y group is independently selected from halo, hydride, (1-6C)alkyl, (1-6C)alkoxy, aryl or aryloxy, either or which is optionally substituted with one or more groups selected from (1-6C)alkyl and halo.

It will be appreciated that the structural formula (III) presented above is intended to show the substituent groups in a clear manner. A more representative illustration of the spatial arrangement of the groups is shown in the alternative representation below:

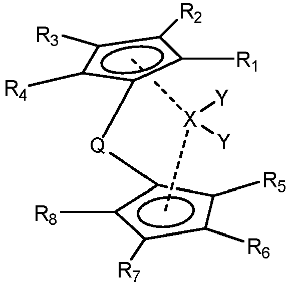

It will also be appreciated that, depending on the identities of substituents R₁-R₈, the compounds of the present invention may be present as *meso* or *rac* isomers, and the present invention includes both such isomeric forms. A person skilled in the art will appreciate that a mixture of isomers of the compound of the present invention may be used for catalysis applications, or the isomers may be separated and used individually (using techniques well known in the art, such as, for example, fractional crystallization).

If the structure of a compound of formula (III) is such that *rac* and *meso* isomers do exist, the compound may be present in the *rac* form only, or in the meso form only.

The catalytic compositions of the invention exhibit superior catalytic performance when compared with current metallocene compounds/compositions used in the polymerisation of α-olefins. In particular, when compared with analogous solid MAO-supported the compositions of the invention, containing a modified solid MAO support material, exhibit significantly increased catalytic activity in the homopolymerisation and copolymerisation of α-olefins.

The compound of formula (III) may be immobilized on the solid phase support material by one or more ionic or covalent interactions.

In the compositions of the invention, the solid-phase support materials of the invention are the only inorganic solid supports used (i.e. no additional solid support such as SiO₂, Al₂O₃ and ZrO₂ are necessary). Moreover, given the dual function of the solid-phase support materials of the invention (as catalytic support and activator species), the catalytic compositions of the invention contain no additional catalytic activator species.

In an embodiment, R₁ and R₂ are each hydrogen.

In another embodiment, R₃ and R₄ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl and (1-6C)alkoxy.

Suitably, R₃ and R₄ are each independently hydrogen or (1-4C)alkyl, or R₃ and R₄ are linked such that, when taken in combination with the atoms to which they are attached, they form a 6-membered fused aromatic ring optionally substituted with one or more groups selected from methyl, ethyl and tert-butyl.

In another embodiment, R₅ and R₆ are each independently hydrogen or (1-4C)alkyl, or R₅ and R₆ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl and (1-6C)alkoxy.

Suitably, R₅ and R₆ are each independently hydrogen or (1-4C)alkyl, or R₅ and R₆ are linked such that, when taken in combination with the atoms to which they are attached, they form a 6-membered fused aromatic ring optionally substituted with one or more groups selected from methyl, ethyl and tert-butyl.

In another embodiment, R₇ and R₈ are each independently hydrogen or (1-4C)alkyl, or R₇ and R₈ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl and (1-6C)alkoxy.

Suitably, R₇ and R₈ are each independently hydrogen or (1-4C)alkyl, or R₇ and R₈ are linked such that, when taken in combination with the atoms to which they are attached, they form a 6-membered fused aromatic ring optionally substituted with one or more groups selected from methyl, ethyl and tert-butyl.

In another embodiment, Q is absent, or is a bridging group selected from -CH₂- or - CH₂CH₂-, either or which may be optionally substituted with one or more groups selected from (1-4C)alkyl and phenyl, or Q is a bridging group -Si(R₉)(R₁₀)-,
wherein Rg and R₁₀ are independently (1-4C)alkyl or aryl.

In another embodiment, X is zirconium.

In another embodiment, each Y group is independently selected from halo.

Suitably, each Y group is chloro.

In another embodiment, the compound of formula (III) has a structure according to either of formulae (IIIa) or (IIIb) shown below: 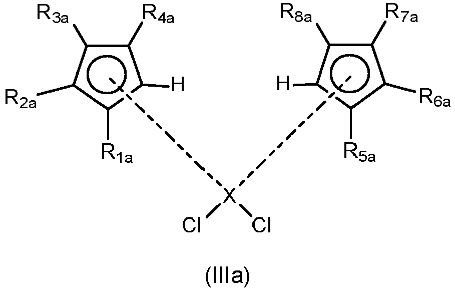 wherein
X, R₁ₐ, R₂ₐ, R₃ₐ, R₄ₐ, R₅ₐ, R₆ₐ, R₇ₐ and R₈ₐ have any of the definitions recited respectively hereinbefore for X, R₁, R₂, R₃, R₄, R₅, R₆, R₇ and R₈ 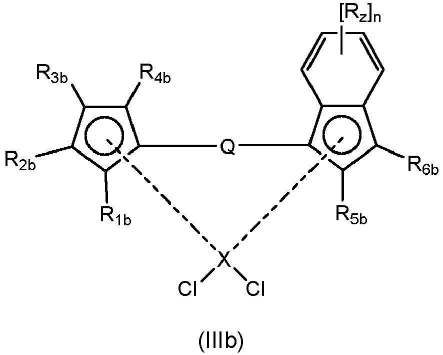 wherein
Q, X, R_{1b}, R_{2b}, R_{3b}, R_{4b}, R_{5b} and R_{6b} have any of the definitions recited respectively hereinbefore for Q, X, R₁, R₂, R₃, R₄, R₅ and R₆;
each R_{z} is independently halo, (1-4C)alkyl or phenyl; and
n is 0, 1, 2, 3 or 4.

In another embodiment, the compound of formula (III) has a structure according to formula (IIIa), wherein X is Zr.

In another embodiment, the compound of formula (III) has a structure according to formula (IIIa), wherein R₁ₐ, R₂ₐ, R₅ₐ and R₆ₐ are each hydrogen.

In another embodiment, the compound of formula (III) has a structure according to formula (IIIa), wherein
R₁ₐ, R₂ₐ, R₅ₐ and R₆ₐ are each hydrogen; and
   i) R₃ₐ and R₇ₐ are independently (1-4C)alkyl and R₄ₐ and R₈ₐ are hydrogen, or
   ii) R₃ₐ and R₄ₐ are linked such that when taken with the carbon atoms to which they are attached they collectively form a 6-membered aryl group that is optionally substituted with one or more groups selected from halo, (1-4C)alkyl and phenyl, and
R₇ₐ and R₈ₐ are linked such that when taken with the carbon atoms to which they are attached they collectively form a 6-membered aryl group that is optionally substituted with one or more groups selected from halo, (1-4C)alkyl and phenyl.

In another embodiment, the compound of formula (III) has a structure according to formula (IIIb), wherein X is Zr.

In another embodiment, the compound of formula (III) has a structure according to formula (IIIb), wherein R_{1b} and R_{2b} are each hydrogen.

In another embodiment, the compound of formula (III) has a structure according to formula (IIIb), wherein R_{3b} and R_{4b} are each hydrogen, or R_{3b} and R_{4b} are linked such that when taken with the carbon atoms to which they are attached they collectively form a 6-membered aryl group that is optionally substituted with one or more groups selected from halo, (1-4C)alkyl and phenyl.

In another embodiment, the compound of formula (III) has a structure according to formula (IIIb), wherein R_{5b} and R_{6b} are each hydrogen, or R_{3b} and R_{4b} are linked such that when taken with the carbon atoms to which they are attached they collectively form a 6-membered aryl group that is optionally substituted with one or more groups selected from halo, (1-4C)alkyl and phenyl.

In another embodiment, the compound of formula (III) has a structure according to formula (IIIb), wherein Q is a bridging group selected from -CH₂- or -CH₂CH₂-, either or which may be optionally substituted with one or more groups selected from (1-4C)alkyl and phenyl, or Q is a bridging group -Si(R₉)(R₁₀)-,
wherein Rg and R₁₀ are independently (1-4C)alkyl or phenyl.

In another embodiment, the compound of formula (III) has a structure according to any of formulae (IIIc), (IIId), (IIIe) or (IIIf) shown below: 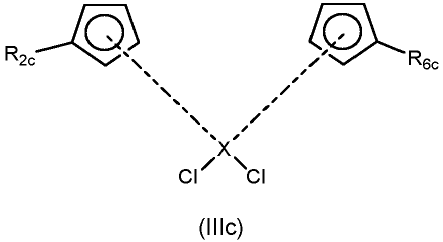 wherein
X, R_{2c} and R_{6c} have any of the definitions recited respectively hereinbefore for X, R₂ and R₆ 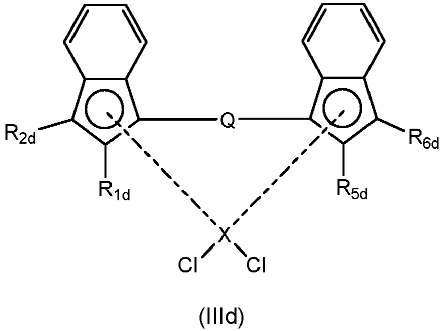 wherein
X, Q, R_{1d}, R_{2d}, R_{5d} and R_{6d} have any of the definitions recited respectively hereinbefore for X, Q, R₁, R₂, R₅ and R₆ 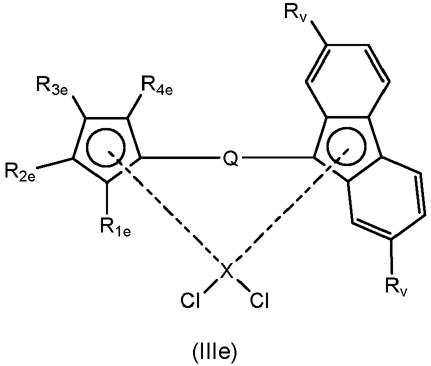 wherein
X, Q, R₁ₑ, R₂ₑ, R₃ₑ and R₄ₑ have any of the definitions recited respectively hereinbefore for X, Q, R₁, R₂, R₃ and R₄; and
each Rᵥ is independently (1-4C)alkyl or phenyl 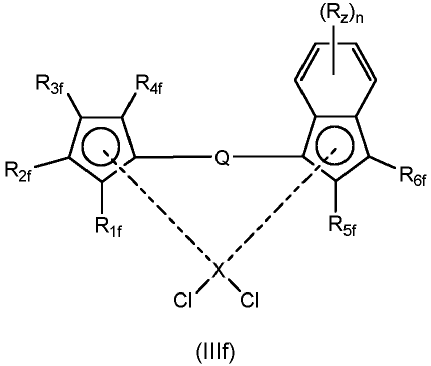
wherein
R_{z}, n, X, Q, R_{1f}, R_{2f}, R_{3f}, R_{4f}, R_{5f} and R_{6f} have any of the definitions recited respectively hereinbefore for R_{z}, n, X, Q, R₁, R₂, R₃, R₄, R₅ and R₆.

In another embodiment, the compound of formula (III) has a structure according to formula (IIIc), wherein R_{2c} and R_{6c} are each independently methyl or n-butyl.

In another embodiment, the compound of formula (III) has a structure according to formula (IIId), wherein Q is absent (in which case each cycopentadienyl ring is bound to hydrogen at this position) or is a bridging group selected from -CH₂- or -CH₂CH₂-, either or which may be optionally substituted with one or more groups selected from (1-4C)alkyl and phenyl, or Q is a bridging group -Si(R₉)(R₁₀)-,
wherein Rg and R₁₀ are independently (1-2C)alkyl or phenyl.

In another embodiment, the compound of formula (III) has a structure according to formula (IIId), wherein R_{1d}, R_{2d}, R_{5d} and R_{6d} are each independently hydrogen or (1-3C)alkyl.

In another embodiment, the compound of formula (III) has a structure according to formula (IIIe), wherein R₁ₑ, R₂ₑ, R₃ₑ and R₄ₑ each independently hydrogen or (1-3C)alkyl.

In another embodiment, the compound of formula (III) has a structure according to formula (IIIe), wherein Q is a bridging group selected from -CH₂- or -CH₂CH₂-, either or which may be optionally substituted with one or more groups selected from (1-4C)alkyl and phenyl, or Q is a bridging group -Si(R₉)(R₁₀)-,
wherein Rg and R₁₀ are independently (1-2C)alkyl or phenyl.

In another embodiment, the compound of formula (III) has a structure according to formula (IIIe), wherein each Rᵥ is independently methyl or tert-butyl

In another embodiment, the compound of formula (III) has a structure according to formula (IIIt), wherein R_{1f}, R_{2f}, R_{3f}, R_{4f}, R_{5f} and R_{6f} are each independently hydrogen or (1-3C)alkyl, and
each R_{z} is independently (1-3C)alkyl.

In another embodiment, the compound of formula (III) has a structure according to formula (IIIf), wherein Q is a bridging group selected from -CH₂- or -CH₂CH₂-, either or which may be optionally substituted with one or more groups selected from (1-4C)alkyl and phenyl, or Q is a bridging group -Si(R₉)(R₁₀)-,
wherein Rg and R₁₀ are independently (1-2C)alkyl or phenyl.

In another embodiment, the compound of formula (III) has any of the structures A-F shown below:

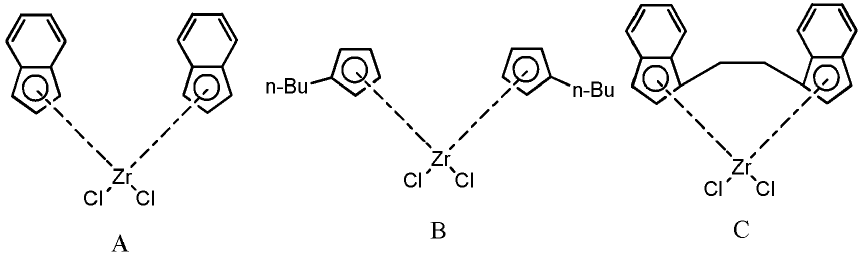

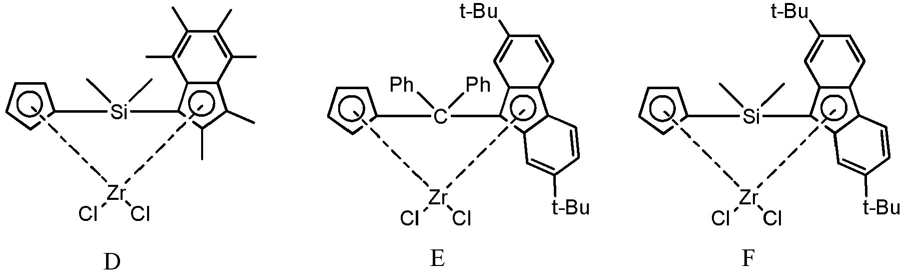

In another embodiment, the compound of formula (III) has any of the structures A-E shown below:

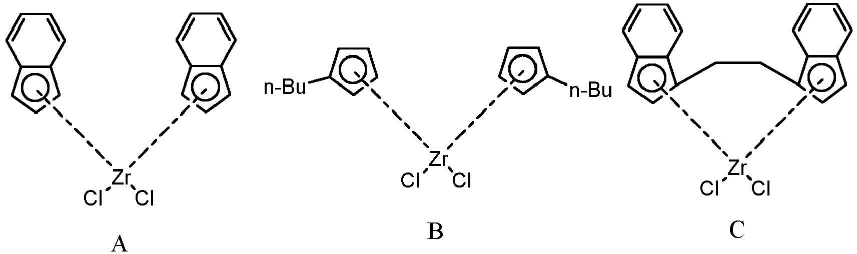

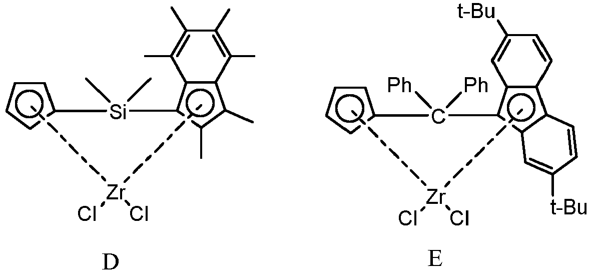

Within the catalytic composition of the invention, any of the compounds according to formula (III) may be provided with any solid-phase support material defined herein.

In a particularly suitable embodiment, when Q (in formula (III)) is ethylene, R_{d} is selected from hydrogen, hydroxyl, (1-4C)alkyl, (2-4C)alkenyl, (2-4C)alkynyl and halo.

### Preparation of catalytic compositions

As described hereinbefore, the present invention also provides a method for the preparation of a catalytic composition defined herein, the method comprising the steps of:
a) providing a solid-phase support material as defined herein in a suitable solvent
b) contacting the solid-phase support material of step a) with a compound of formula (III) defined herein; and
c) isolating the product resulting from step b).

The solid-phase support materials of the invention are straightforwardly prepared using mild reaction conditions.

Suitable solvents for use in step a) will be well known to one of ordinary skill in the art, and include toluene, o-xylene, mesitylene, pentane, hexane, heptane, cyclohexane and methylcyclohexane. Suitably, the solvent used in step a) is toluene.

Step b) may involve mixing the reagents for a period of 0.05-6 hours. Step b) may be conducted at a temperature of 1-3 hours.

### Applications

As discussed hereinbefore, the present invention also provides a use of the solid-phase support material defined herein as a solid-phase support for supporting a metallocene catalytic compound.

The solid-phase support materials of the invention present a number of advantages over other solid-phase support material. Perhaps most notably, when used in the preparation of supported metallocene-based Ziegla-Natta catalysts for use in the polymerisation of olefins, the solid-phase support materials of the invention give rise to a marked increase in catalytic activity.

In an embodiment, the solid-phase support material is usued as a solid-phase support for supporting a metallocene catalytic compound in an olefin (e.g. ethene) polymerisation reaction.

As discussed hereinbefore, the present invention also provides a use of a catalytic composition as defined herein as a polymerisation catalyst for the preparation of a polyolefin.

As discussed hereinbefore, the present invention also provides a process for forming a polyolefin (e.g. a polyethylene), the process comprising the step of reacting olefin monomers in the presence of a catalytic composition defined herein.

The catalytic compositions of the invention exhibit superior catalytic performance when compared with current metallocene compounds/compositions used in the polymerisation of α-olefins. In particular, when compared with analogous solid MAO-supported the compositions of the invention, containing a modified solid MAO support material, exhibit significantly increased catalytic activity in the homopolymerisation and copolymerisation of α-olefins.

The following paragraphs describe preferred embodiments of the above-described use and method aspects of the invention.

Since the only inorganic solid support employed in the catalytic compositions of the invention is the solid-phase support material of the invention (i.e. no additional solid support such as SiO₂, Al₂O₃ and ZrO₂ are necessary), the compositions may be used in the absence of an inorganic support material. Moreover, given the dual function of the solid-phase support materials of the invention (as catalytic support and activator species), the catalytic compositions of the invention may be used in the absence of an additional catalytic activator species.

In an embodiment, the polyolefin is polyethylene. In such embodiments, the olefin monomers are ethene molecules.

In another embodiment, the polyolefin is a copolymer formed from ethene monomers comprising 1-10 wt%, by total weight of the monomer, of one or more (4-8C) α-olefin. Suitably, the (4-8C) α-olefin is 1-butene, 1-hexene, 1-octene, or a mixture thereof.

A person skilled in the art of olefin polymerization will be able to select suitable reaction conditions (e.g. temperature, pressures, reaction times etc.) for such a polymerization reaction. A person skilled in the art will also be able to manipulate the process parameters in order to produce a polyolefin having particular properties.

### EXAMPLES

One or more non-limiting examples of the invention will now be described, for the purpose of illustration only, with reference to the accompanying figures, in which
Fig. 1: SEM images (x250 magnification) of PE samples from catalyst based on solid MAO (a) un-modified, and modified via Method A with 2 mol% loading of (b) B(C₆F₅)₃,
Fig. 2: SEM image (x250 magnification) of polyethylene samples from a catalyst based on solid MAO modified with 20 mol% B(C₆F₅)₃ via Method A.
Fig. 3: SEM images (x4000 magnification) of solid MAO samples from (a) control, and modified with (b) 10 mol% B(C₆F₅)₃, and (c) 40 mol% C₆F₅OH via Method B.
Fig. 4: SEM of modified solid MAO with modifier pentafluorophenol: Al ratio of a) 0.0005:1, b) 0.4:1 and c) 1.2:1.
Fig. 5: SEM of modified solid MAO with modifier 4-fluorophenol: Al ratio of a) 0.0005:1.
Fig. 6: SEM of modified solid MAO with modifier phenol: Al ratio of a) 0.0005:1.
Fig. 7: SEM of modified solid MAO with modifier 4-fluorophenylboronic acid: Al ratio of a) 0.0005:1.
Fig. 8: SEM of modified solid MAO with modifier p-toluene sulfonamide: Al ratio of a) 0.0005:1.
Fig. 9: SEM images (x250 magnification) of PE samples from catalysts based on (a)-(d) unmodified and (e)-(h) 5 mol% B(C₆F₅)₃ modified solid MAO with 4 zirconocene complexes.
Fig. 10: SEM images (x1000 magnification) of PE samples from catalysts based on (a)-(b) unmodified and (c)-(d) 5 mol% C₆F₅OH modified solid MAO with 2 different zirconocene complexes.
Fig. 11: Selected region of the ¹H NMR spectrum in *d*₈-THF of solid MAO modified with BPh₃ at 5 mol% loading via Method B.
Fig. 12: Selected region of the ¹H NMR spectrum in *d*₈-THF of solid MAO modified with B(C₆F₅)₃ at 5 mol% loading via Method B.
Fig. 13: ¹⁹F{¹H} NMR spectrum in *d*₈-THF of solid MAO modified with B(C₆F₅)₃ at 5 mol% loading via Method B.
Fig. 14: Selected region of the ¹H NMR spectrum in *d*₈-THF of solid MAO modified with Al(C₆F₅)₃ at 5 mol% loading via Method B.
Fig. 15: ¹⁹F{¹H} NMR spectrum in *d*₈-THF of solid MAO modified with Al(C₆F₅)₃ at 5 mol% loading via Method B.
Fig. 16: Selected region of the ¹H NMR spectrum in *d*₈-THF of solid MAO modified with {4-F}C₆H₄B(OH)₂ at 5 mol% loading via Method B.
Fig. 17: Selected region of the ¹H NMR spectrum in *d*₈-THF of solid MAO modified with {3,5-F}₂C₆H₃B(OH)₂ at 5 mol% loading via Method B.
Fig. 18: ¹⁹F{¹H} NMR spectrum in *d*₈-THF of solid MAO modified with {3,5-F}₂C₆H₃B(OH)₂ at 5 mol% loading via Method B.
Fig. 19: Selected region of the ¹H NMR spectrum in *d*₈-THF of solid MAO modified with C₆F₅B(OH)₂ at 5 mol% loading via Method B.
Fig. 20: Selected region of the ¹H NMR spectrum in *d*₈-THF of solid MAO modified with {4-F}C₆H₄OH at 5 mol% loading via Method B.
Fig. 21: ¹⁹F{¹H} NMR spectrum in *d*₈-THF of solid MAO modified with {4-F}C₆H₄OH at 5 mol% loading via Method B.
Fig. 22: Selected region of the ¹H NMR spectrum in *d*₈-THF of solid MAO modified with {3,5-F}₂C₆H₃OH at 5 mol% loading via Method B.
Fig. 23:¹⁹F{¹H} NMR spectrum in *d*₈-THF of solid MAO modified with {3,5-F}₂C₆H₃OH at 5 mol% loading via Method B.
Fig. 24: Selected region of the ¹H NMR spectrum in *d*₈-THF of solid MAO modified with C₆F₅OH at 5 mol% loading via Method B.
Fig. 25: ¹⁹F{¹H} NMR spectrum in *d*₈-THF of solid MAO modified with C₆F₅OH at 5 mol% loading via Method B.
Fig. 26: ¹¹B DEPTH SSNMR spectrum (15 kHz spinning) of 5 mol% BPh₃ modified solid MAO via Method B.
Fig. 27: ¹⁹F DEPTH SSNMR spectrum (15 kHz spinning) of 20 mol% B(C₆F₅)₃ modified solid MAO via Method B.
Fig. 28: ¹¹B DEPTH SSNMR spectrum (10 kHz spinning) of 20 mol% B(C₆F₅)₃ modified solid MAO via Method B.
Fig. 29: ¹⁹F{¹H} DEPTH SSNMR spectrum (24 kHz spinning) of 5 mol% Al(C₆F₅)₃ modified solid MAO via Method B.
Fig. 30: ¹⁹F{¹H} DEPTH SSNMR spectrum (10 kHz spinning) of 5 mol% {4-F}C₆H₄B(OH)₂ modified solid MAO via Method B.
Fig. 31: ¹¹B DEPTH SSNMR spectrum (24 kHz spinning) of 5 mol% {4-F}C₆H₄B(OH)₂ modified solid MAO via Method B.
Fig. 32: ¹⁹F{¹H} DEPTH SSNMR spectrum (10 kHz spinning) of 5 mol% {3,5-F}₂C₆H₃B(OH)₂ modified solid MAO via Method B.
Fig. 33: ¹¹B{¹⁹F}{¹H} DEPTH SSNMR spectrum (24 kHz spinning) of 5 mol% {3,5-F}₂C₆H₃B(OH)₂ modified solid MAO via Method B.
Fig. 34: ¹⁹F{¹H} DEPTH SSNMR spectrum (24 kHz spinning) of 5 mol% C₆F₅B(OH)₂ modified solid MAO via Method B.
Fig. 35: ¹¹B{¹⁹F}{¹H} DEPTH SSNMR spectrum (24 kHz spinning) of 5 mol% C₆F₅B(OH)₂ modified solid MAO via Method B.
Fig. 36: ¹⁹F{¹H} DEPTH SSNMR spectrum (15 kHz spinning) of 10 mol% {4-F}C₆H₄OH modified solid MAO via Method B.
Fig. 37: ¹⁹F{¹H} DEPTH SSNMR spectrum (24 kHz spinning) of 5 mol% {3,5-F}₂C₆H₃OH modified solid MAO via Method B.
Fig. 38: ¹⁹F{¹H} DEPTH SSNMR spectrum (15 kHz spinning) of 10 mol% C₆F₅OH modified solid MAO via Method B.
Fig. 39: DRIFT spectrum (NaCl window) of solid MAO modified with BPh₃ at 5 mol% loading via Method B.
Fig. 40: DRIFT spectrum (NaCl window) of solid MAO modified with B(C₆F₅)₃ at 5 mol% loading via Method B.
Fig. 41: DRIFT spectrum (NaCl window) of solid MAO modified with {4-F}C₆H₄B(OH)₂ at 5 mol% loading via Method B.
Fig. 42: DRIFT spectrum (NaCl window) of solid MAO modified with {3,5-F}₂C₆H₃B(OH)₂ at 5 mol% loading via Method B.
Fig. 43: DRIFT spectrum (NaCl window) of solid MAO modified with C₆F₅B(OH)₂ at 5 mol% loading via Method B.
Fig. 44: DRIFT spectrum (NaCl window) of solid MAO modified with {4-F}C₆H₄OH at 5 mol% loading via Method B.
Fig. 45: DRIFT spectrum (NaCl window) of solid MAO modified with {3,5-F}₂C₆H₃OH at 5 mol% loading via Method B.
Fig. 46: DRIFT spectrum (NaCl window) of solid MAO modified with C₆F₅OH at 5 mol% loading via Method B.

### Example 1 - Preparation of solid MAO

The solid MAO useful in the preparation of the solid-phase support material of the invention may be prepared *via* an adaptation of the optimised procedure in Kaji *et al.* in the US 8,404,880 B2 embodiment 1 (Scheme 1). For brevity, each synthesised solid MAO is represented as solid MAO(Step 1 Al:O ratio/Step 2 temperature in °C,time in h/Step 3 temperature in °C,time in h). Hence, the synthesis conditions outlined in Scheme 1 would yield solid MAO(1.2/70,32/100,12).

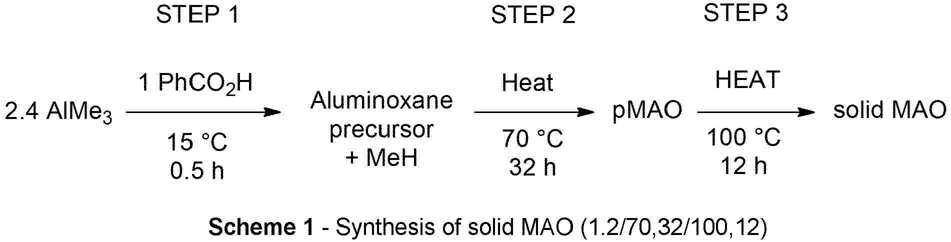

A Rotaflo ampoule containing a solution of trimethyl aluminium (2.139 g, 2.967 mmol) in toluene (8 mL) was cooled to 15 °C with rapid stirring, and benzoic acid (1.509 g, 1.239 mmol) was added under a flush of N₂ over a period of 30 min. Effervescence (presumably methane gas, MeH) was observed and the reaction mixture appeared as a white suspension, which was allowed to warm to room temperature. After 30 min the mixture appeared as a colourless solution and was heated in an oil bath at 70 °C for 32 h (a stir rate of 500 rpm was used). The mixture obtained was a colourless solution free of gelatinous material, which was subsequently heated at 100 °C for 12 h. The reaction mixture was cooled to room temperature and hexane (40 mL) added, resulting in the precipitation of a white solid which was isolated by filtration, washed with hexane (2 x 40 mL) and dried *in vacuo* for 3 h. Total yield = 1.399 g (71% based on 40 wt% Al).

### Example 2 - Preparation of solid-phase support materials

### Method A

Typical experiment: To a Schlenk flask charged with solid MAO (Example 1) and the modifier was added toluene, and the resulting dispersion was heated at 80 °C for 2 h with regular swirling. The mixture was cooled to room temperature and the insoluble solids were allowed to settle. The supernatant solution was removed by decantation and the remaining slurry was washed three times with a 2:1 mixture of hexane:toluene and dried *in vacuo* overnight, to afford the modified solid MAO as a free-flowing solid.

### Method B

Typical experiment: To a Schlenk flask charged with a dispersion of solid MAO (Example 1) in toluene was added a solution of the modifier in toluene and the flask was sonicated in a water bath at ambient temperature for 1 h. The resultant mixture was allowed to settle, the supernatant solution was removed by decantation and the remaining slurry was washed three times with a 2:1 mixture of hexane:toluene and dried *in vacuo* overnight, to afford the modified solid MAO as a free-flowing solid.

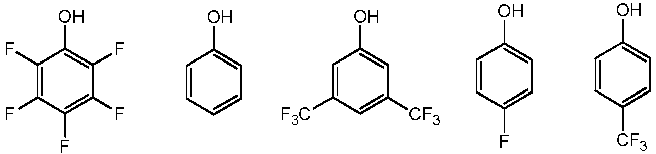

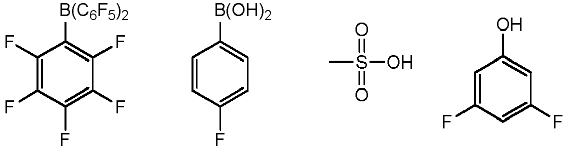

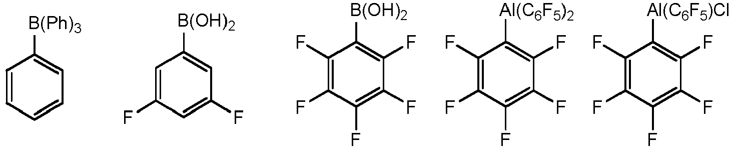

### Example 3 - Characterisation of solid-phase support materials and Polymerisation studies

In order to assess the catalytic performance of the sold-phase support materials, a range of metallocene compounds were supported on a variety of the solid-phase support materials. In a typical experiment, the metallocene catalytic compound (e.g rac-ethylenebis(1-indenyl) zirconium dichloride, (EBI)ZrCl₂) and the support (modified solid MAO) were loaded into a Schlenk flask and toluene (40 mL) was added. The mixture was heated at 80 °C in an oil bath and swirled regularly to ensure complete immobilisation of the metallocene complex (stirring was avoided to prevent aggregation). After 2 h the reaction was removed from the oil bath and allowed to settle, before decantation of the colourless supernatant and thorough drying *in vacuo.*

Once prepared, the ability of the supported metallocene catalysts to catalyse the polymerisation of ethylene to polyethylene was assessed. In a typical polymerisation experiment, the immobilised catalyst (10 mg), tri*iso*butlyaluminium scavenger (150 mg), and hexane (40 mL) were added to a high-pressure Rotaflo ampoule. Ethylene gas was continuously fed into the ampoule at 2 bar overpressure during polymerisation at 70 °C. After 30 min, the reaction was stopped by removing the ampoule from the oil bath, and degassing *in vacuo.* The polymer was isolated on a frit, washed with pentane (50 mL) and vacuum dried at room temperature for 1 h. Each polymerisation experiment was conducted at least twice to ensure the reproducibility of the corresponding outcome, and mean productivities are quoted in units of kg_{PE}g_{CAT}⁻¹ h⁻¹.

For large scale polymerisation studies, polymerization of ethylene was performed in a 2L stainless steel autoclave reactor as the lines of nitrogen and ethylene gases were directly connected into the reactor. First, to clear up the system, the reactor was evacuated and purged by inert nitrogen gas for 1 hour and dried n-hexane was filled. Then, triethylaluminum (TEA) was fed by pipette into the reactor following by finished catalyst, which was prepared in a glove box into the specified sampling catalyst vessel under nitrogen atmosphere. After that, 3 barg of nitrogen was replaced with ethylene for 3 times and kept within the reactor at 3 barg before starting stirrer. When the reactor was heated until temperature below the set point for 5°C, catalyst was injected into the reactor by ethylene pressure flushed with dried n-hexane. Ethylene gas was introduced via a mass flow controller to fulfill the reactor reaching to the set point of total pressure at 8 barg and the temperature was also controlled at 80°C. Polymerisation was continued for 1 hour, while feeding rate was recorded. Lastly, reaction was cut down by depressurised and cooled down temperature. Resulting mixture was poured into tray and dried at room temperature to remove solvent from polymer.

### Supported (EBl)ZrCl₂ complexes

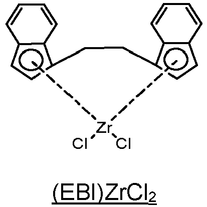

Table 1 below shows characterisation and polymerisation data for solid MAO samples modified via Method A with B(C₆F₅)₃.

**Table 1. Characterisation and polymerisation data for solid MAO samples modified via Method A with B(C₆F₅)₃ Al(C₆F₅)₃ and Al(C₆F₅)₂Cl. Polymerisation conditions: 2 bar, 50 mL hexanes, TIBA.**

| Modifier | Ratio M:Al mole | Support % Yield | Support BET /m²mmol_{Al}⁻¹ | Support wt% Al | Catalyst Activity /kg_{PE}mol_{Zr}⁻¹h⁻¹ |
|---|---|---|---|---|---|
| Control | 0 | 88 | 17.8 | 40.0 | 13686 |
| B(C₆F₅)₃ | 0.02 | 87 | 15.0 | 32.6 | 13972 |
| Al(C₆F₅)₃ | 0.02 | 83 | X | 30.9 | 12670 |
| Al(C₆F₅)₂Cl | 0.02 | 80 | X | 32.5 | 11461 |

Although the Al-based modifiers gave rise to support materials having activities that are slightly lower than the control, it is presumed that this is attributable to the relatively high temperature employed in Method A. It is expected that such modifiers will yield improved results when the support material is prepared according to Method B, which uses softer conditions. Preliminary studies support this hypothesis.

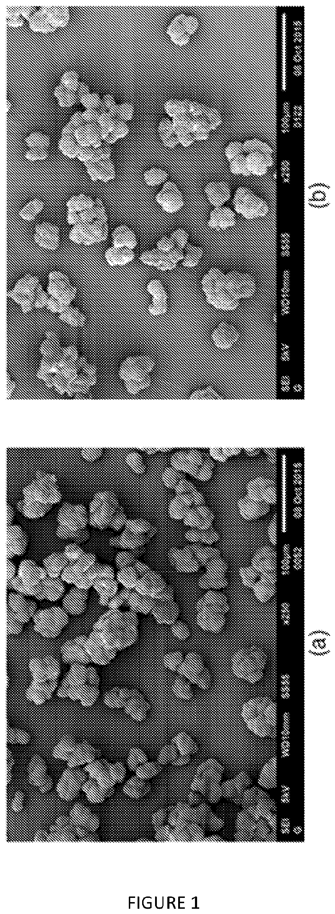

Fig. 1 shows SEM images of polymer samples on carbon tape, which demonstrate that that the PE particle size and morphology is not significantly affected by B(C₆F₅)₃ modified support (Fig. 1b) with respect to the control (Fig. 1a).

Table 2 below shows characterisation and polymerisation data for solid MAO samples modified via Method A with increased amounts of B(C₆F₅)₃ (compared with the data of Table 1).

**Table 2. Characterisation and polymerisation data for solid MAO samples modified via Method A with B(C₆F₅)₃ at increased loadings. Polymerisation conditions: 2 bar, 50 mL hexanes, TIBA**

| Ratio M:Al mole | Support % Yield | Support BET /m² mmol_{Al}⁻¹ | Support wt% Al | Catalyst Activity /kg_{PE}mol_{Zr}⁻¹h⁻¹ |
|---|---|---|---|---|
| 0 | 88 | 17.8 | 40.0 | 13686 |
| 0.01 | 88 | 14.6 | 35.3 | 10787 |
| 0.02 | 87 | 15.0 | 34.9 | 11533 |
| 0.04 | 58 | 10.6 | 23.0 | 11739 |
| 0.10 | 59 | 12.8 | 23.5 | 14028 |
| 0.20 | 48 | 9.6 | 19.0 | 14402 |

Although the modifier loadings of 0.01, 0.02 and 0.04 resulted in catalyst activities that are slightly lower than the control, it is expected that this is attributable to the relatively high temperature employed in Method A. It is expected that such loadings will yield improved results when the support material is prepared according to Method B, which uses softer conditions. Preliminary studies support this hypothesis. It is also noted that the results illustrated in Table 1 demonstrate that B(C₆F₅)₃ loadings of 0.02 may nonetheless give rise to improved activities when the support material is prepared via Method A.

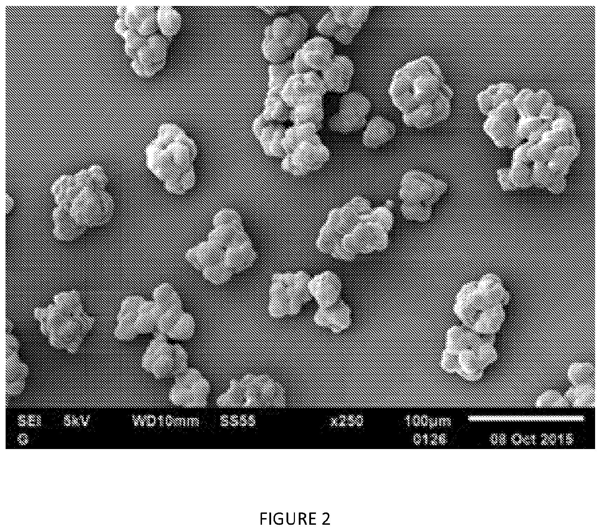

SEM imaging (Fig. 2) confirmed that the PE particle size and morphology is preserved with the 20 mol% B(C₆F₅)₃ modified catalyst.

Table 3 below shows characterisation and polymerisation data for solid MAO samples modified via Method B with two different modifiers, B(C₆F₅)₃ and C₆F₅OH.

**Table 3. Characterisation and polymerisation data for solid MAO samples modified via Method B with (EBl)ZrCl₂. Polymerisation conditions: 8 bar, 1000 mL hexanes, TEA.**

| Modifier | Ratio M:Al mole | Support % Yield | Support wt% Al | Catalyst Activity /kg_{PE} mol_{Zr}⁻¹ h⁻¹ |
|---|---|---|---|---|
| Control | 0 | 93.2 | 35.1 | 143608 |
| B(C₆F₅)₃ | 0.10 | 72.9 | 24.8 | 211559 |
| C₆F₅OH | 0.40 | 98.8 | 18 | 262977 |

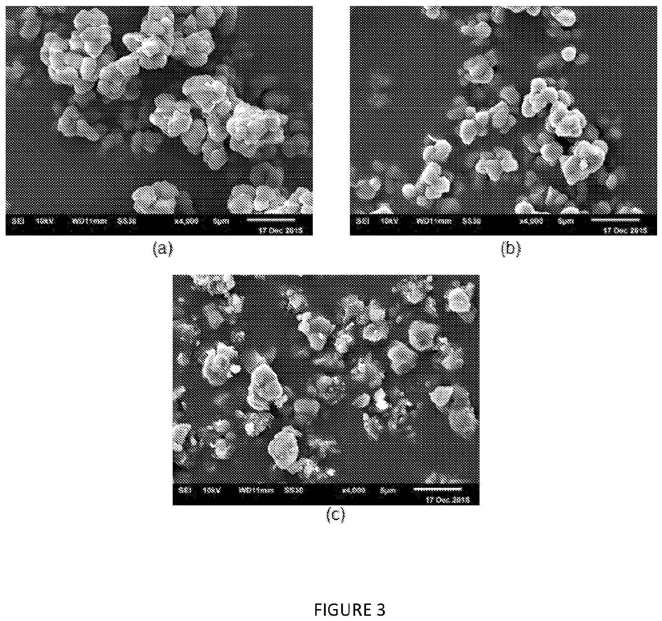

SEM images of the solid MAO samples mounted on copper tape (Fig. 3) reveal that the particle size and morphology is not significantly affected by B(C₆F₅)₃ and C₆F₅OH modified supports with respect to the control.

Table 4 below shows characterisation and polymerisation data for solid MAO samples modified at 10 mol% loading via Method B with two different modifiers, B(C₆F₅)₃ and C₆F₅OH.

**Table 4. Characterisation and polymerisation data for solid MAO samples modified at 10 mol% loading via Method B with (EBl)ZrCl₂ Polymerisation conditions: 2 bar, 50 mL hexanes, TIBA.**

| Modifier | Ratio M:Al mole | Support Colour | Support % Yield | Support wt.% Al | Support BET /m²mmol_{Al}⁻¹ | Catalyst Activity /kg_{PE}mol_{Zr}⁻¹h⁻¹ |
|---|---|---|---|---|---|---|
| Control | 0 | Colourless | 88 | 39.7 | 15.0 | 12515 |
| B(C₆F₅)₃ | 0.10 | Colourless | 62 | 24.9 | 18.6 | 16009 |
| {4-F}C₆H₄OH | 0.10 | Orange | 92 | 36.7 | 12.0 | 11366 |
| {4-CF₃}C₆H₄OH | 0.10 | Green | 87 | 34.8 | 14.9 | 6891 |
| {3,5-F}₂C₆H₃OH | 0.10 | Grey | 93 | 37.0 | 14.7 | 9178 |
| {3,5-CF₃}₂C₆H₃OH | 0.10 | Dark red | 95 | 37.9 | 11.9 | 3945 |
| C₆F₅OH | 0.10 | Colourless | 92 | 36.5 | 14.4 | 13340 |

Table 5 shows polyethylene polymerisation data for solid MAO modified with varying quantities of pentafluorophenol via Method B.

**Table 5: Summary data of modified solid MAO with pentafluorophenol (Al wt%) and modified solidMAO/(EBl)ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*w | MWD |
|---|---|---|---|---|---|---|---|
| | | Al | Zr | | | | |
| control | 36 | 36 | 0.4 | 18.8 | 0.38 | 180,109 | 4.30 |
| 0.01 | 27 | 27 | 0.3 | 32.8 | 0.44 | 186,827 | 4.78 |
| 0.05 | 24 | 24 | 0.3 | 34.2 | 0.44 | 182,812 | 4.42 |
| 0.1 | 21 | 21 | 0.4 | 32.0 | 0.37 | 192,566 | 4.47 |
| 0.4 | 17 | 17 | 0.3 | 57.9 | 0.38 | 147,578 | 3.88 |
| 0.8 | 15 | 15 | 0.5 | 16.8 | 0.37 | 158,497 | 4.11 |
| 1.2 | 10 | 10 | 0.3 | 19.7 | 0.33 | 193,992 | 4.59 |

| | | | | | | | |
|---|---|---|---|---|---|---|---|
| Polymerisation conditions: 25 mg Catalyst, 2.5 mL TEA, 80 °C, 8 bar. 1 L hexanes | | | | | | | |

The aluminum content (wt%) of modified solid MAO decreases from 27, 24, 21 to 17 when more modifier was used. It indicates the presence of phenol compound in modified product corresponding to amount of phenol addition. At ratio of 0.4 it performs 3 folds higher catalyst activity [activity: 18.8 vs 57.9 ×10⁴ Kg_{PE}/mol_{Zr}·h] compared with non-modified solid MAO/(EBl)ZrCl₂. Bulk density of obtained polymer is in the range of 0.33 - 0.44 ml/g which is acceptable for polymerization.

### Supported (^{nBu}Cp)₂ZrCl₂ complexes

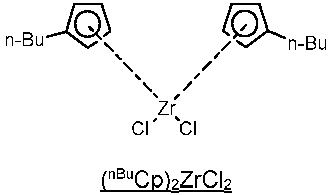

Table 6 shows polyethylene polymerisation data for solid MAO modified with varying quantities of pentafluorophenol via Method B.

**Table 6: Summary data of modified solidMAO with pentafluorophenol (Al wt%) and modified solid MAOl(^{nBu}Cp)₂ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*w | MWD |
|---|---|---|---|---|---|---|---|
| | | Al | Zr | | | | |
| control | 36 | 40 | 0.8 | 31.2 | 0.34 | 142,466 | 2.54 |
| 0.0005 | 40 | 34 | 0.6 | 38.2 | 0.35 | 138,051 | 2.54 |
| 0.001 | 35 | 35 | 0.6 | 34.9 | | | |
| 0.01 | 27 | 33 | 0.4 | 53.7 | 0.39 | 139,519 | 2.51 |
| 0.05 | 24 | 33 | 0.4 | 40.9 | | | |
| 0.1 | 21 | 37 | 0.5 | 30.7 | | | |
| 0.4 | 17 | 30 | 0.3 | 36.9 | | | |
| 0.8 | 15 | 14 | 0.2 | 53.1 | | | |
| 1.2 | 10 | 12 | 0.3 | 37.6 | | | |
| 1.6 | 9 | 12 | 0.2 | 45.9 | | | |

| | | | | | | | |
|---|---|---|---|---|---|---|---|
| Polymerisation conditions: 25 mg Catalyst, 2.5 mL TEA, 80 °C, 8 bar. 1 L hexanes | | | | | | | |

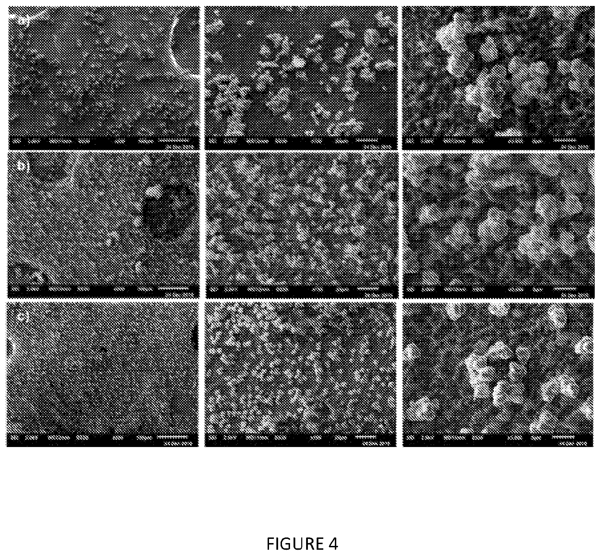

The increase of modifier amount (modifier: Al ratio of 0.0005 to 1.6) results in lower Al content (wt%) of modified solid MAO and Al & Zr (wt%) content in modified solid MAO/(^{nBu}Cp)₂ZrCl₂. At ratio of 0.01 it shows the highest activity of 53.7 × 10⁴ Kg_{PE}/mol_{Zr}·h which is higher than non-modified solid MAO/(^{nBu}Cp)₂ZrCl₂ of 31.2 x 10⁴ Kg_{PE}/mol_{Zr}·h. The bulk density of polymer obtained is in the range of 0.35 - 0.39 ml/g which is acceptable for slurry polymerization. The modification of solid MAO has no significant effect to polymer properties such as molecular weight and molecular weight distribution as shown in Table 6. The morphology of modified solid MAO still well controls. They have popcorn shape with particle size of 4-7 micron (Fig. 4).

Table 7 shows polyethylene polymerisation data for solid MAO modified with 4-fluorophenol via Method B.

**Table 7: Summary data of modified solidMAO with 4-fluorophenol (Al wt%) and modified solidMAO/(^{nBu}Cp)₂ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*w | MWD |
|---|---|---|---|---|---|---|---|
| | | Al | Zr | | | | |
| control | 39 | 40 | 0.8 | 31.2 | 0.34 | 142,466 | 2.54 |
| 0.0005 | 40 | - | - | - | | | |
| 0.05 | 40 | 37 | 0.49 | 29.83 | | | |

| | | | | | | | |
|---|---|---|---|---|---|---|---|
| Polymerisation conditions: 12.5 mg Catalyst, 2.5 mL TEA, 80 °C, 8 bar. 1 L hexanes. | | | | | | | |

Table 8 shows polyethylene polymerisation data for solid MAO modified with phenol via Method B.

**Table 8: Summary data of modified solid MAO with phenol (Al wt%) and modified solid MAO/(^{nBu}Cp)₂ZrCl₂ (Al wt%, Zr wt%, activity, productivity) and polymer properties (bulk density, M_{w}, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*w | MWD |
|---|---|---|---|---|---|---|---|
| | | Al | Zr | | | | |
| control | 39 | 40 | 0.8 | 31.2 | 0.34 | 142,466 | 2.54 |
| 0.0005 | 35 | 36 | 0.56 | 37.75 | | | |

| | | | | | | | |
|---|---|---|---|---|---|---|---|
| Polymerisation conditions: 12.5 mg Catalyst, 2.5 mL TEA, 80 °C, 8 bar. 1 L hexanes. | | | | | | | |

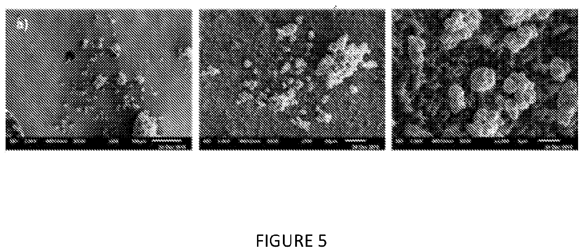

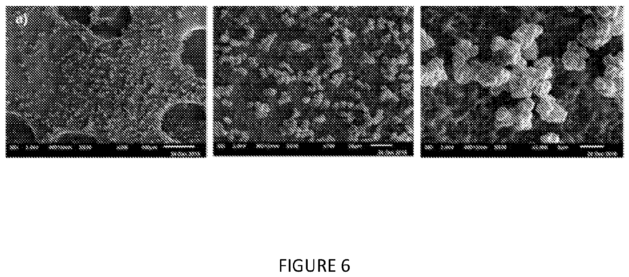

Higher amount of modifier addition (higher modifier:Al) provides lower Al and Zr content in modified solid MAO and modified solid MAO/(^{nBu}Cp)₂ZrCl₂. The morphology of modified solid MAO was elucidated with SEM, (Fig. 5 and 6).

Table 9 shows polyethylene polymerisation data for solid MAO modified with methanesulfonic acid via Method B

**Table 9: Summary data of modified solid MAO with methanesulfonic acid (Al wt%) and modified solid MAO/(^{nBU}Cp)₂ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | 4 Activity ×10⁴ (Kg_{PE}/mol_{Zr}·h) | Bulk density (ml/g) | *M*w | MWD |
|---|---|---|---|---|---|---|---|
| | | Al | Zr | | | | |
| control | 39 | 40 | 0.8 | 31.2 | 0.34 | 142,466 | 2.54 |
| 0.0045 | 36 | 36 | 0.48 | 33.8 | 0.35 | | |

| | | | | | | | |
|---|---|---|---|---|---|---|---|
| Polymerisation conditions: 12.5 mg Catalyst, 2.5 mL TEA, 80 °C, 8 bar. 1 L hexanes. | | | | | | | |

The modifier:Al of 0.0045 shows higher activity of 33.8x 10⁴ KgP_{E}/mol_{Zr}·h with lower Zr content of 0.48 wt%. Bulk density is 0.35 ml/g acceptable for slurry polymerization.

Table 10 shows polyethylene polymerisation data for solid MAO modified with 4-fluorophenylboronic acid via Method B

**Table 10: Summary data of modified solid MAO with 4-fluorophenylboronic acid (Al wt%) and modified solid MAO/(^{nBU}Cp)₂ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*w | MWD |
|---|---|---|---|---|---|---|---|
| | | Al | Zr | | | | |
| control | 39 | 40 | 0.8 | 31.2 | 0.34 | 142,466 | 2.54 |
| 0.0005 | 33 | 36 | 0.48 | 33.1 | 0.30 | | |

| | | | | | | | |
|---|---|---|---|---|---|---|---|
| Finish catalyst 0.0125 g, TEA (200 mmol/L) = 2.5 mL, T = 80°C, P = 8 Bar, solvent hexane 1 L | | | | | | | |

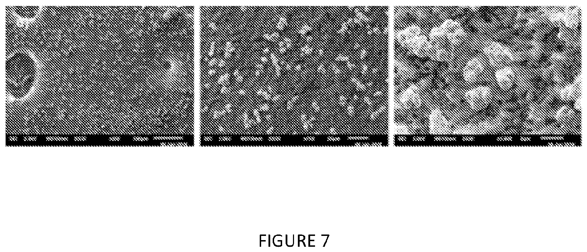

The modifier:Al of 0.0005 showed an increased activity of 33.1×10⁴ Kg_{PE}/mol_{Zr}·h. Fig. 7 shows the morphology of the modified solid MAO.

Table 11 shows polyethylene polymerisation data for solid MAO modified with 3,5-Bis(trifluoromethane)phenol via Method B

**Table 11: Summary data of modified solid MAO with 3,5-Bis(trifluoromethane)phenol (Al wt%) and modified solidMAO/(^{nBu}Cp)₂ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*_{w} | MWD |
|---|---|---|---|---|---|---|---|
| | | Al | Zr | | | | |
| control | 39 | 40 | 0.8 | 31.2 | 0.34 | 142,466 | 2.54 |
| 0.0006 | 34 | 38 | 0.51 | 34.6 | | | |

| | | | | | | | |
|---|---|---|---|---|---|---|---|
| Polymerisation conditions: 12.5 mg Catalyst, 2.5 mL TEA, 80 °C, 8 bar. 1 L hexanes. | | | | | | | |

Table 12 shows polyethylene polymerisation data for solid MAO modified with p-toluene sulfonamide via Method B.

**Table 12: Summary data of modified solidMAO with p-toluene sulfonamide (Al wt%) and modified solidMAO/(^{nBu}Cp)₂ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) | Bulk density (ml/g) | *M*_{w} | MWD |
|---|---|---|---|---|---|---|---|
| | | Al | Zr | | | | |
| control | 39 | 40 | 0.8 | 31.2 | 0.34 | 142,466 | 2.54 |
| 0.0005 | 32 | 36 | 0.58 | 28.40 | | | |

| | | | | | | | |
|---|---|---|---|---|---|---|---|
| Polymerisation conditions: 12.5 mg Catalyst, 2.5 mL TEA, 80 °C, 8 bar. 1 L hexanes. | | | | | | | |

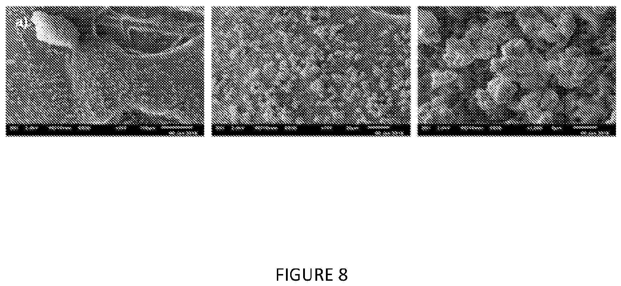

Fig. 8 shows the morphology of the p-toluene sulfonamide-modified solid MAO at 0.0005 mol ratio.

### Supported (Ind)₂ZrCl₂ complexes

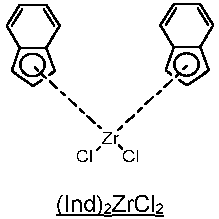

Table 13 shows polyethylene polymerisation data for solid MAO modified with pentafluorophenol via Method B.

**Table 13: Summary data of modified solid MAO with pentafluorophenol (Al wt%) and modified solidMAO/(Ind)₂ZrCl₂ (Al wt%, Zr wt%, activity, productivity) and polymer properties (bulk density, Mw, MWD).**

| Ratio M:Al (mole) | Al (wt%) - ICP Modified solid MAO | Finish catalyst ICP (wt%) | | Activity ×10⁴ (Kg_{PE}/mol_{Zr}·h) | Bulk density (ml/g) | *M*_{w} | MWD |
|---|---|---|---|---|---|---|---|
| | | Al | Zr | | | | |
| control | 36 | 41 | 0.78 | 18.47 | 0.34 | 142,466 | 2.54 |
| 0.0005 | 40 | | | | 0.32 | | |
| 0.4 | 17 | 19 | 0.44 | 28.4 | | | |

| | | | | | | | |
|---|---|---|---|---|---|---|---|
| Polymerisation conditions: 25 mg Catalyst, 2.5 mL TEA, 80 °C, 8 bar. 1 L hexanes. | | | | | | | |

The modification of solid MAO with pentafluorophenol with modifier:Al ratio of 0.4 used as solid activator for (Ind)₂ZrCl₂ is able to enhance in catalyst activity of 28.4 Kg_{PE}/gCat·h.

### Supported ^{Ph2}C(^{tBu}Flu,Cp)ZrCl₂ complexes

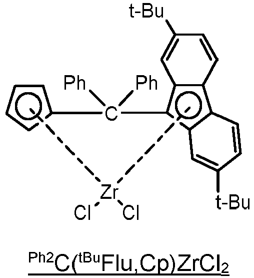

Table 14 shows polyethylene polymerisation data for solid MAO modified with varying quantities pf pentafluorophenol via Method B.

**Table 14: Summary data of modified solid MAO with pentafluorophenol (Al wt%) and modified solid MAO/^{Ph2}C(^{tBU}Flu,Cp)ZrCl₂ (Al wt%, Zr wt%, activity, productivity) and polymer properties (bulk density, Mw, MWD).**

| Ratio M:Al (mole) | Al (wt%) - ICP Modified solidMAO | Finish catalyst ICP (wt%) | | Activity ×10⁴ (Kg_{PE}/mol_{Zr}·h) |
|---|---|---|---|---|
| | | Al | Zr | |
| control | 36 | 38 | 0.58 | 5.3 |
| 0.0005 | 40 | 36 | 0.6 | 6.2 |
| 0.001 | 35 | 38 | 0.67 | 5.5 |
| 0.01 | 27 | 37 | 0.67 | 5.4 |
| 0.05 | 24 | 30 | 0.34 | 11.6 |
| 0.1 | 21 | 29 | 0.29 | 15.9 |
| 0.4 | 17 | 16 | 0.36 | 9.2 |
| 0.8 | 15 | 12 | 0.40 | 7.7 |
| 1.2 | 10 | 11 | 0.13 | 3.2 |
| 1.6 | 9 | 11 | 0.08 | 1.0 |

| | | | | |
|---|---|---|---|---|
| Polymerisation conditions: 25 mg Catalyst, 2.5 mL TEA, 80 °C, 8 bar. 1 L hexanes | | | | |

### Further studies

Table 15 shows characterisation and polymerisation data for solid MAO samples modified with B(C₆F₅)₃ via Method B at different loadings, and polymerisation activities with 2 different zirconocene precatalysts.

**Table 15. Characterisation data for solid MAO samples modified with B(C₆F₅)₃ via Method B at different loadings, and polymerisation activities with 2 different zirconocene precatalysts.**

| Ratio M : Al (mol) | Support Yield (%) | Support ICP-MS (wt% Al) | Support BET (m²mmol_{Al}⁻¹) | Activity (kg_{PE}mol_{Zr}⁻¹h⁻¹) | |
|---|---|---|---|---|---|
| | | | | (EBl)ZrCl₂ | (^{nBu}Cp)₂ZrCl₂ |
| 0.00 | 94 | 38.5 | 16.6 | 12692 | 11900 |
| 0.01 | 89 | 38.1 | 14.2 | 15662 | 10827 |
| 0.05 | 78 | 36.4 | 15.2 | 16718 | 9690 |
| 0.10 | 62 | 32.5 | 16.8 | 16106 | 9346 |
| 0.20 | 44 | 25.3 | 11.6 | 17834 | 9088 |

Table 15 shows the polymerisation activities using (EBl)ZrCl₂ and (^{nBu}Cp)₂ZrCl₂ when B(C₆F₅)₃ was used as modifier at different molar ratios. This demonstrates an increase with the former but a decrease with the latter.

Table 16 shows characterisation and polymerisation data for solid MAO samples modified with C₆F₅OH via Method B at different loadings, and polymerisation activities with 2 different zirconocene precatalysts.

**Table 16. Characterisation data for solid MAO samples modified with C₆F₅OH via Method B at different loadings, and polymerisation activities with 2 different zirconocene precatalysts.**

| Ratio M : Al (mol) | Support Yield (%) | Support ICP-MS (wt% Al) | Support BET (m²mmol_{Al}⁻¹) | Activity (kg_{PE}mol_{Zr}⁻¹h⁻¹) | |
|---|---|---|---|---|---|
| | | | | (EBl)ZrCl₂ | (^{nBu}Cp)₂ZrCl₂ |
| 0.00 | 94 | 38.5 | 16.6 | 12692 | 11900 |
| 0.01 | 93 | 37.5 | 15.6 | 12500 | 8119 |
| 0.05 | 83 | 36.8 | 14.8 | 13330 | 9099 |
| 0.10 | 91 | 34.4 | 14.4 | 13023 | 7655 |
| 0.20 | 93 | 25.4 | 11.9 | 14392 | 5153 |

Table 16 shows the polymerisation activities using (EBl)ZrCl₂ and (^{nBu}Cp)₂ZrCl₂ when C₆F₅OH was used as modifier at different molar ratios. This demonstrates an increase with the former but a decrease with the latter.

Tables 17 to 19 shows the molecular weights of the polyethylene using various modifiers on solid MAO and various complexes.

**Table 17. Polymerisation Activities and GPC data for unmodified solid MAO (control) with 5 different zirconocene precatalysts.**

| Precatalyst | Activity (kg_{PE}mol_{Zr}⁻¹h⁻¹) | M_{w} (kg/mol) | PDI |
|---|---|---|---|
| (EBl)ZrCl₂ | 12692 | 99.0 | 3.5 |
| (^{nBu}Cp)₂ZrCl₂ | 11900 | 254.8 | 2.7 |
| ^{Me2}SB(^{*t*Bu2}Flu,l*)ZrCl₂ | 5849 | 585.6 | 2.8 |
| ^{Me2}SB(Cp,l*)ZrCl₂ | 9468 | 178.2 | 2.7 |

**Table 18. Polymerisation Activities and GPC data for B(C₆F₅)₃ modified solid MAO at 5 mol% loading via Method B with 5 different zirconocene precatalysts.**

| Precatalyst | Activity (kg_{PE}mol_{Zr}⁻¹h⁻¹) | M_{w} (kg/mol) | PDI |
|---|---|---|---|
| (EBl)ZrCl₂ | 17512 | 101.6 | 4.0 |
| (^{nBu}Cp)₂ZrCl₂ | 9690 | 289.9 | 2.6 |
| ^{Me2}SB(^{*t*Bu2}Flu,l*)ZrCl₂ | 6319 | 633.6 | 3.0 |
| ^{Me2}SB(Cp,l*)ZrCl₂ | 7020 | 252.1 | 2.5 |

**Table 19. Polymerisation activities and GPC data for C₆F₅OH modified solid MAO at 5 mol% loading via Method B with 5 different zirconocene precatalysts.**

| Precatalyst | Activity (kg_{PE}mol_{Zr}⁻¹h⁻¹) | M_{w} (kg/mol) | PDI |
|---|---|---|---|
| (EBl)ZrCl₂ | 13330 | 132.9 | 3.7 |
| (^{nBu}Cp)₂ZrCl₂ | 9090 | 290.6 | 2.7 |
| ^{Me2}SB(^{*t*Bu2}Flu,l*)ZrCl₂ | 8016 | 662.2 | 3.3 |
| ^{Me2}SB(Cp,l*)ZrCl₂ | 7671 | 185.5 | 3.2 |

The results presented in Tables 17-19 show that the modification of the support has no direct effect on the molecular weight of the resulting polyethylene.

Table 20 shows characterisation data for solid MAO samples modified with various modifiers at 5 mol% loading via Method B, and polymerisation data with 3 different zirconocene precatalysts.

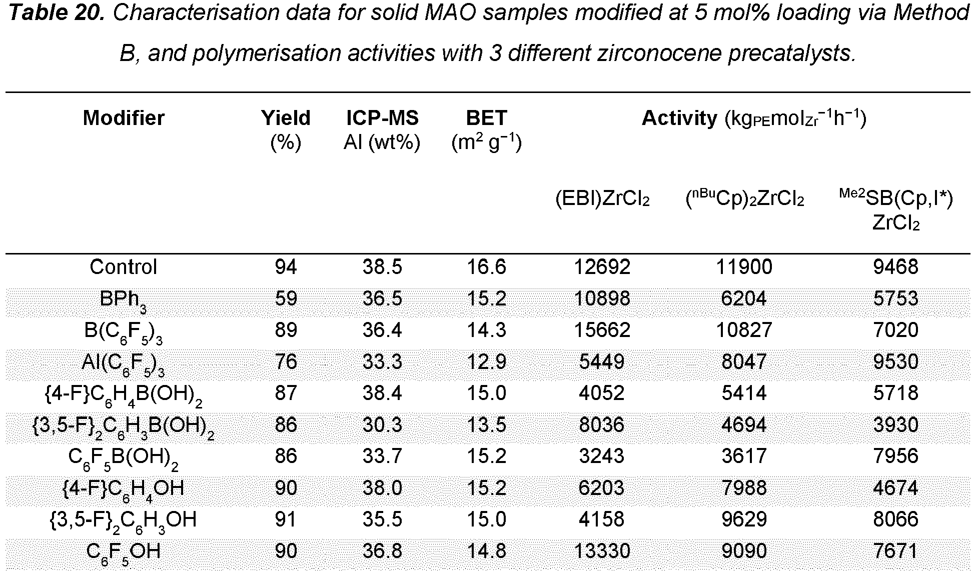

Table 21 shows spectroscopic data for the different modified solid polymethylaluminoxane and complexes.

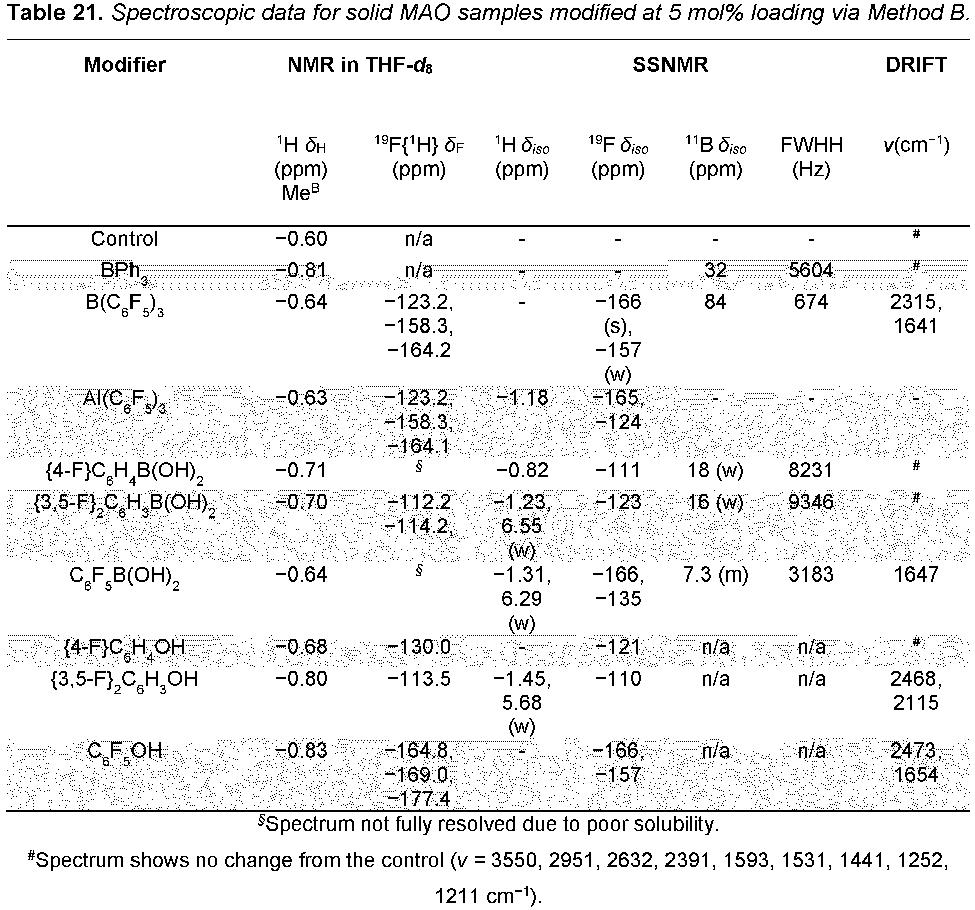

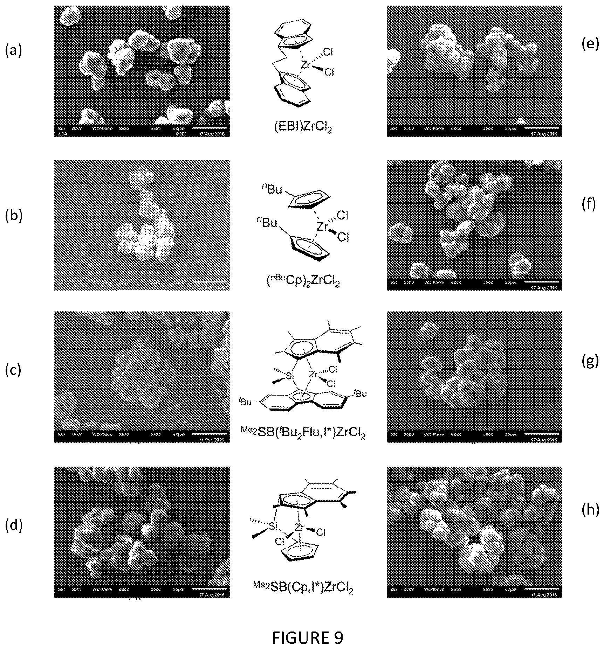

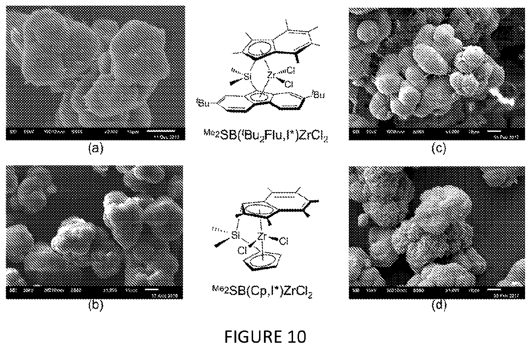

Figures 9 and 10 shows the microscopic images for various polyethylene using various modifiers and zirconocene complexes demonstrating that the shape of the polyethylene remains the same

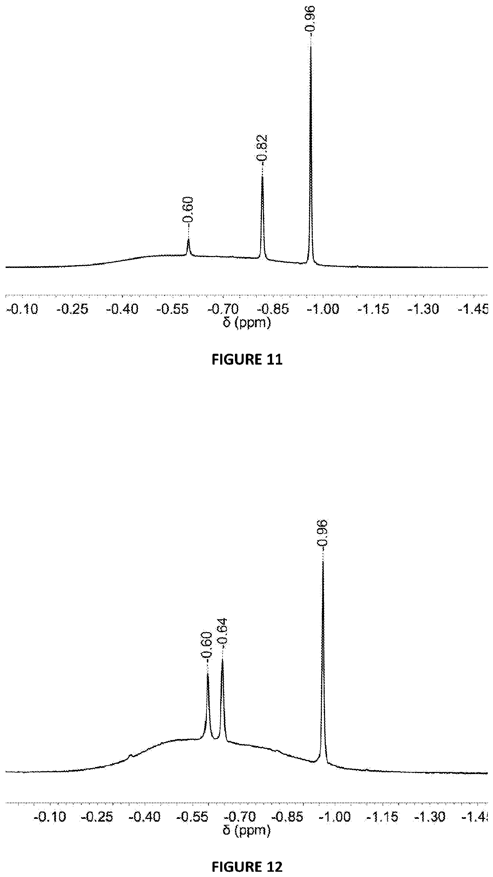

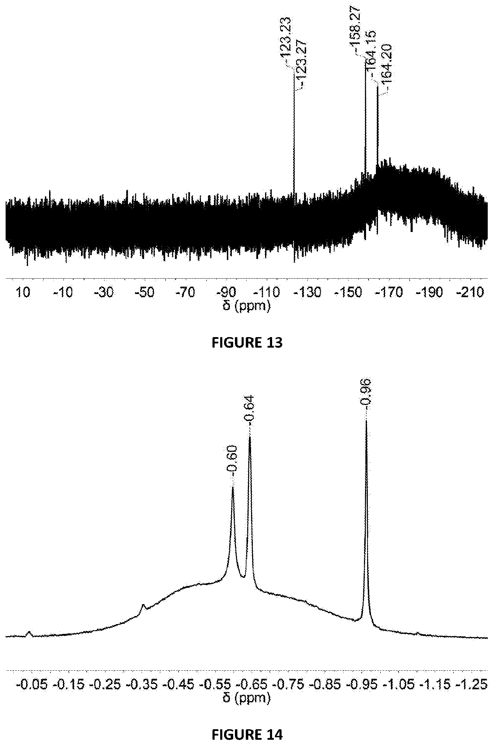

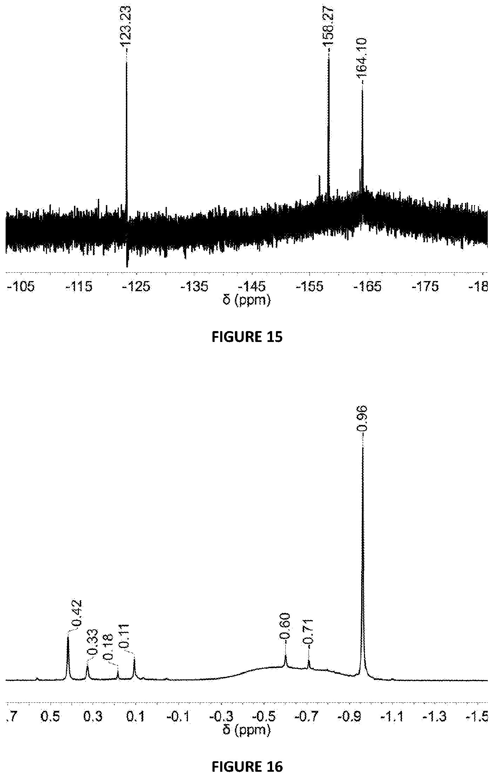

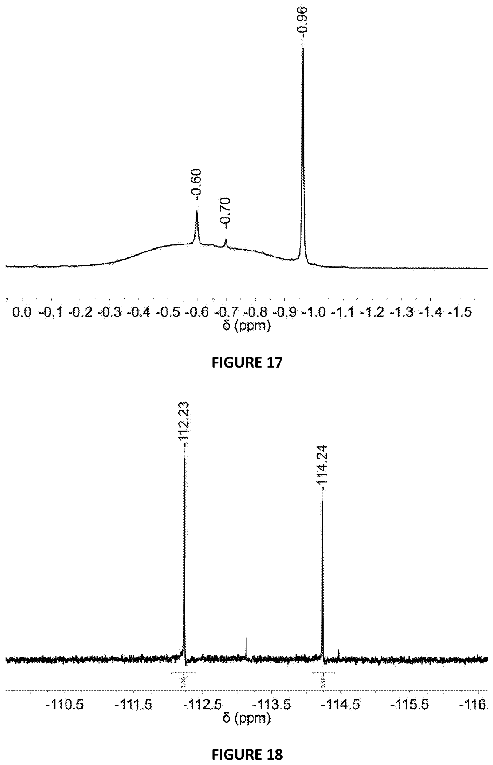

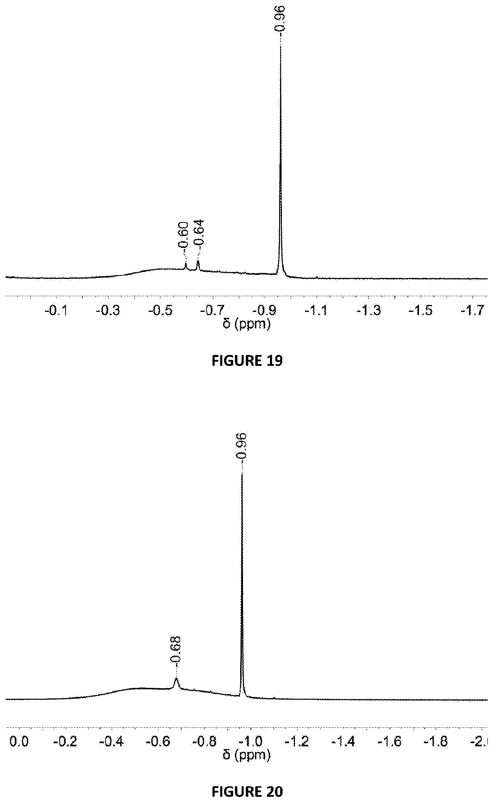

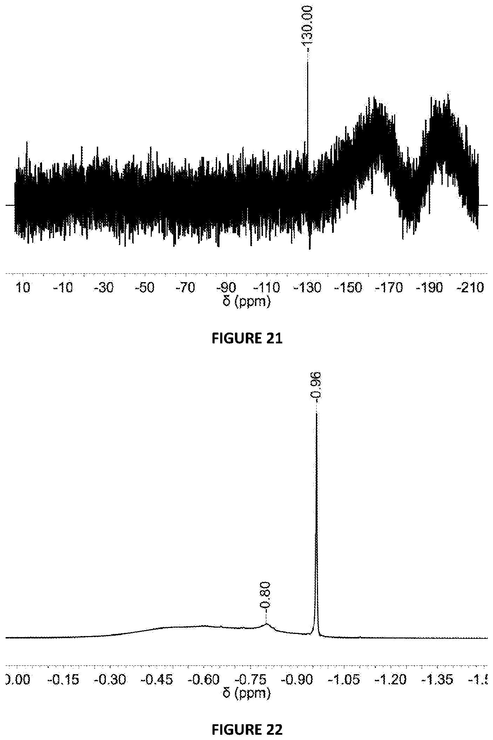

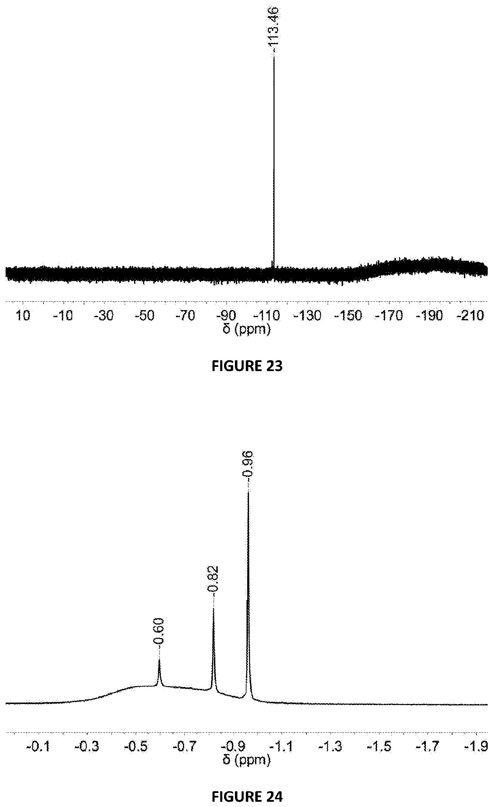

Figures 11 to 25 show the solution NMR spectra of various modified solid polymethylaluminoxane demonstrating the incorporation of the modifier into the product.

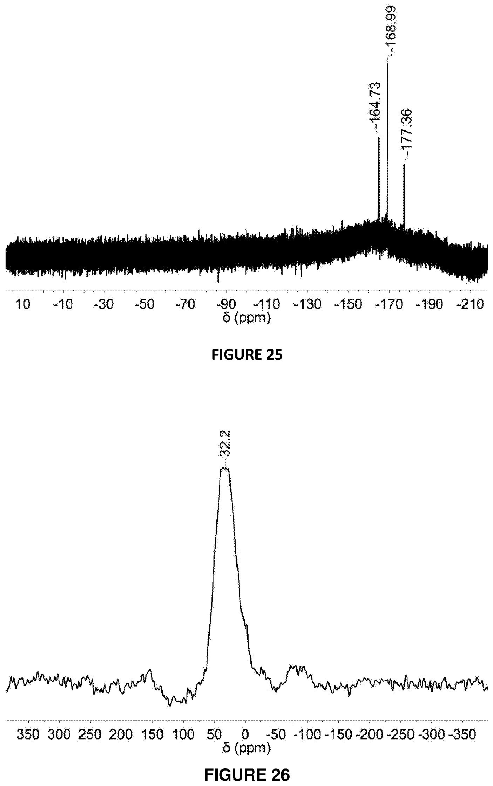

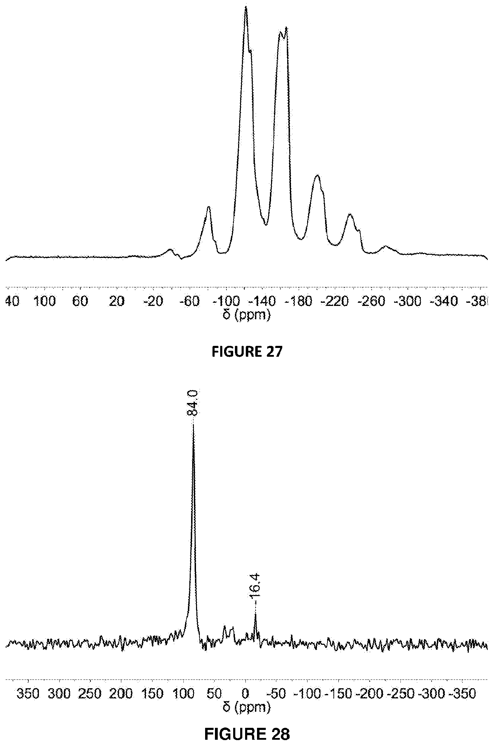

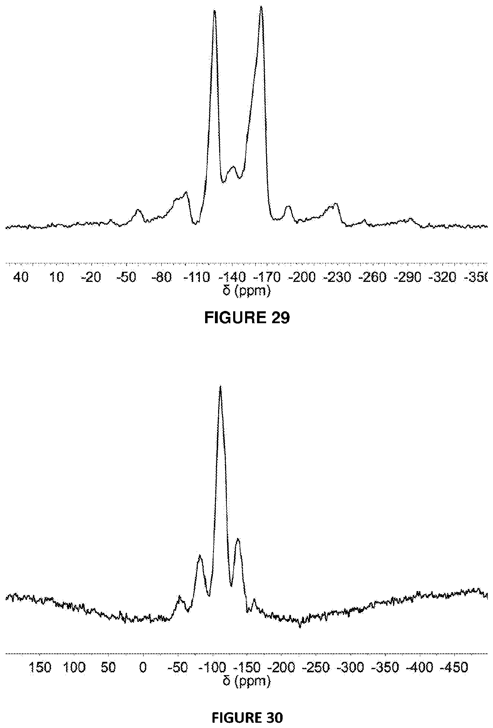

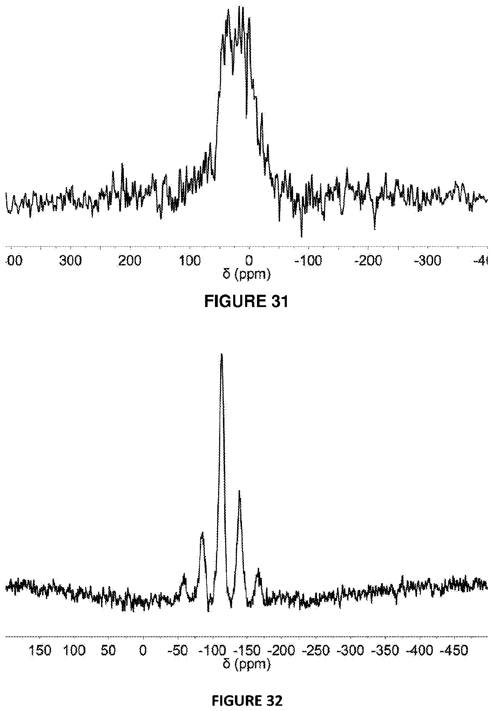

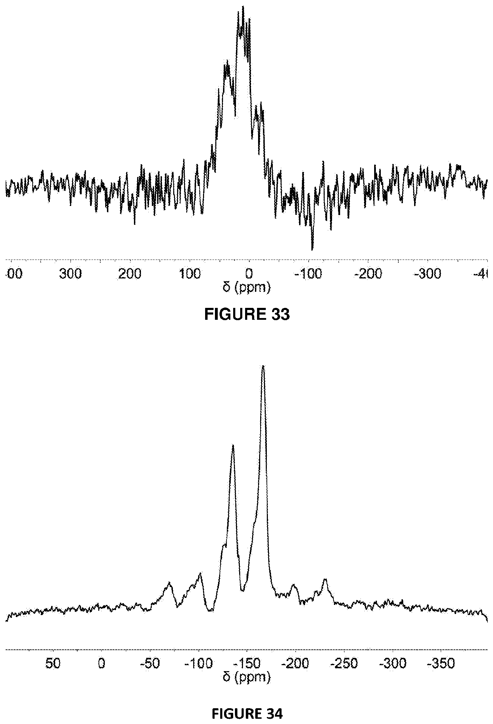

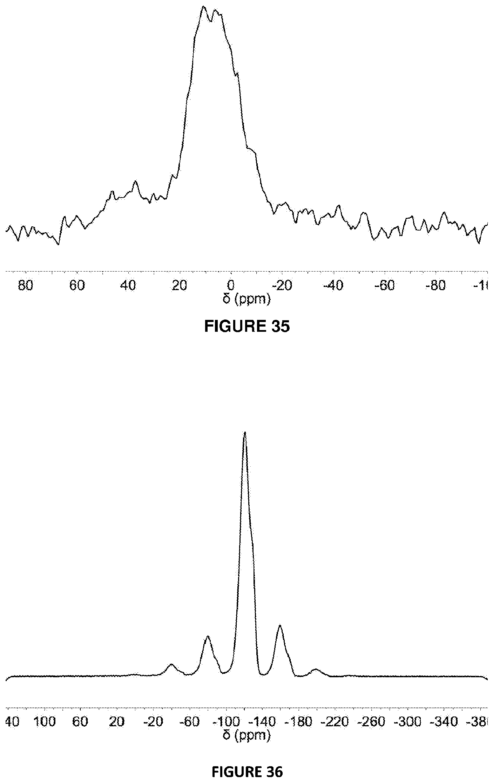

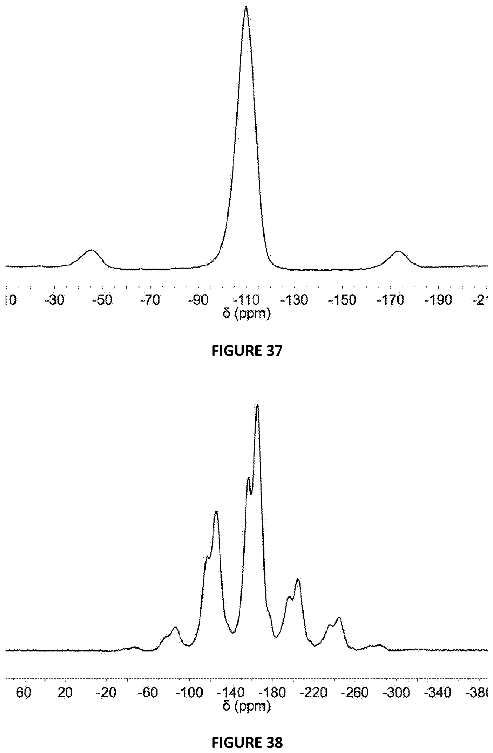

Figures 26 to 38 show the solid state NMR spectra of various modified solid polymethylaluminoxane demonstrating the incorporation of the modifier into the product.

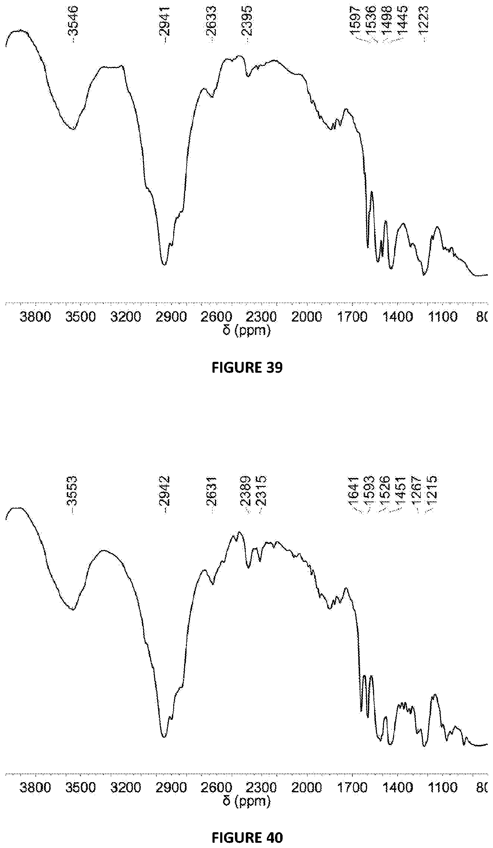

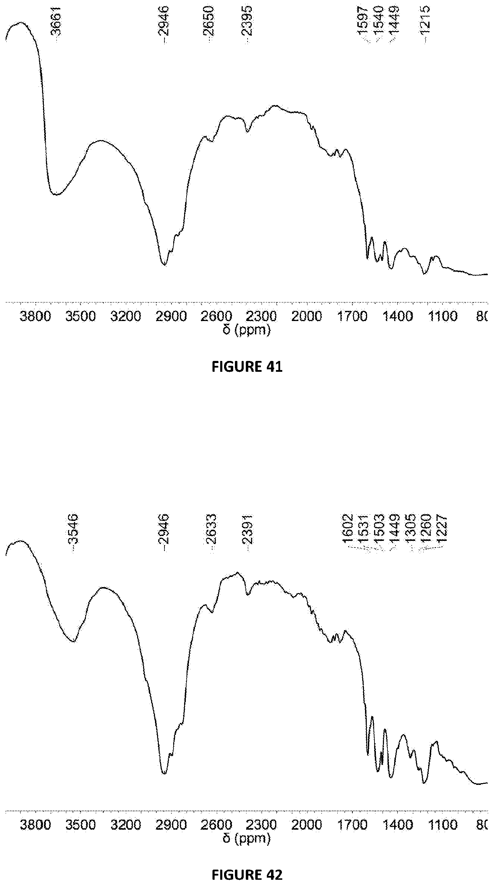

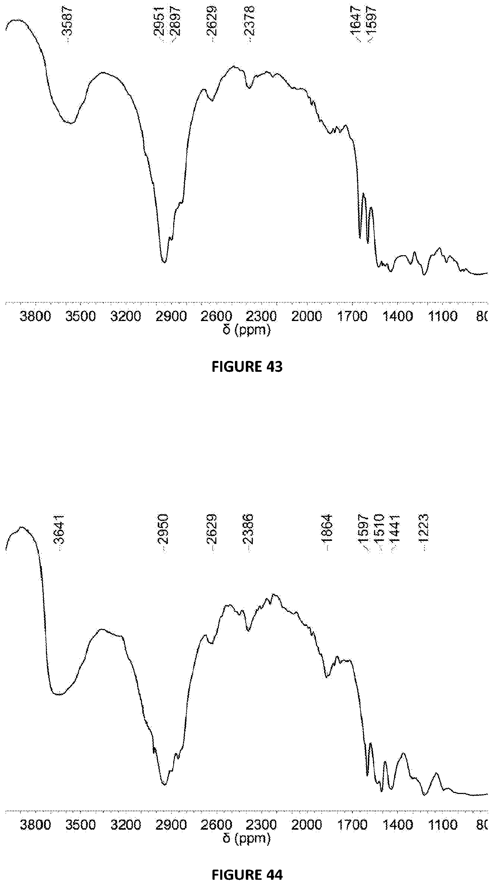

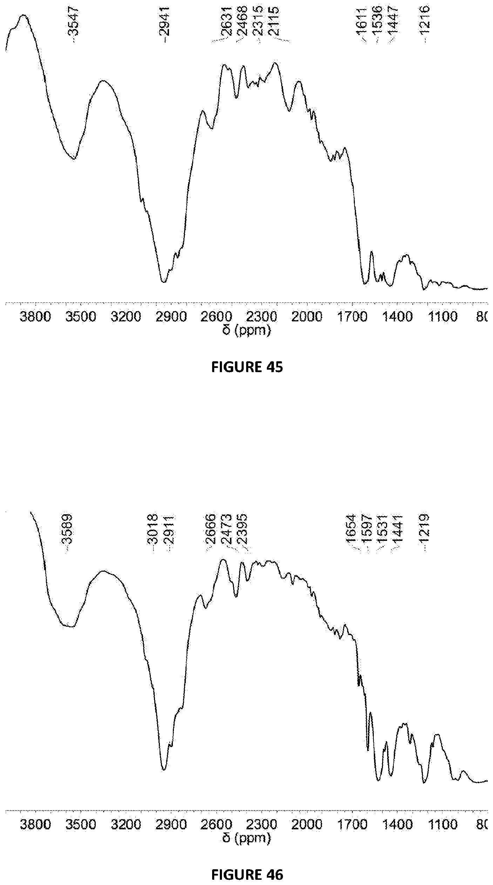

Figures 39 to 46 show the DRIFT spectra of various modified solid polymethylaluminoxane demonstrating the incorporation of the modifier into the product.

While specific embodiments of the invention have been described herein for the purpose of reference and illustration, various modifications will be apparent to a person skilled in the art without departing from the scope of the invention as defined by the appended claims.

## Claims

1. A solid-phase support material suitable for supporting a metallocene catalytic compound, the solid-phase support material comprising a solid polymethylaluminoxane modified by reaction with a compound of formula (I) or (II) shown below: 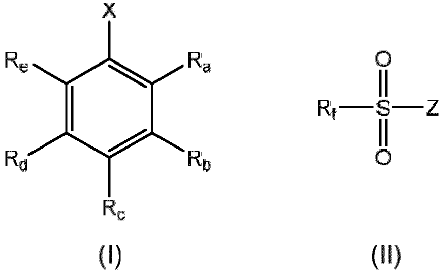 wherein
X is hydroxyl or a group B(Y)₂ or a group Al(Y)₂,
wherein each Y is independently selected from hydroxyl, phenyl and naphthalenyl, any of which may be optionally substituted with one or more groups selected from halo, hydroxyl, (1-4C)alkyl, (2-4C)alkenyl, (2-4C)alkynyl and trihaloalkyl;
Rₐ and R_{b} are independently hydrogen, hydroxyl, (1-4C)alkyl, (2-4C)alkenyl, (2-4C)alkynyl, halo or trihaloalkyl, or
Rₐ and R_{b} are linked, such that, when taken with the atoms to which they are attached, they form a 6-membered aromatic ring that is optionally substituted by one or more groups selected from halo, 1-4C)alkyl, (2-4C)alkenyl, (2-4C)alkynyl and trihaloalkyl;
R_{c}-Rₑ are independently hydrogen, hydroxyl, (1-4C)alkyl, (2-4C)alkenyl, (2-4C)alkynyl, halo or trihaloalkyl;
Z is hydroxyl or a group -NRₓR_{y},
wherein Rₓ and R_{y} are independently selected from hydrogen and (1-4C)alkyl;
R_{f} is (1-4C)alkyl, (2-4C)alkenyl, (2-4C)alkynyl, trihaloalkyl or a phenyl group that is optionally substituted with one or more groups selected from hydroxyl, (1-4C)alkyl, (2-4C)alkenyl, (2-4C)alkynyl, halo and trihaloalkyl;
wherein the mole ratio of the compound of formula (I) or (II) to the solid polymethylaluminoxane within the solid-phase support material ranges from 0.0001:1 to 0.3:1.

2. The solid-phase support material of claim 1, wherein Z is hydroxyl.

3. The solid-phase support material of claim 1 or 2, wherein when X is hydroxyl
i) Rₐ-Rₑ are each independently halo; or
ii) Rₐ and R_{b} are linked, such that, when taken with the atoms to which they are attached, they form a 6-membered aromatic ring that is optionally substituted by one or more groups selected from halo, 1-4C)alkyl, (2-4C)alkenyl, (2-4C)alkynyl and trihaloalkyl, and
R_{c}-Rₑ are independently hydrogen, hydroxyl, (1-4C)alkyl, (2-4C)alkenyl, (2-4C)alkynyl, halo or trihaloalkyl; or
iii) Rₐ, R_{b} and Rₑ are each independenlty hydrogen, hydroxyl, (1-4C)alkyl, (2-4C)alkenyl, (2-4C)alkynyl, halo or trihaloalkyl, and
R_{d} and Rₑ are each independently hydrogen, hydroxyl, (1-4C)alkyl, (2-4C)alkenyl, (2-4C)alkynyl or trihaloalkyl; or
iv) Rₐ, R_{b}, R_{d} and Rₑ are each independently hydrogen, hydroxyl, (1-4C)alkyl, (2-4C)alkenyl, (2-4C)alkynyl, halo or trihaloalkyl, and
R_{c} is hydrogen, hydroxyl, (1-4C)alkyl, (2-4C)alkenyl, (2-4C)alkynyl or halo.

4. The solid-phase support material of claim 1, 2 or 3, wherein any one or more of the following statements (A) to (C) applies:
(A) each Y is independently selected from hydroxyl or a phenyl group that is optionally substituted with one or more groups selected from fluoro, hydroxyl, (1-4C)alkyl, (2-4C)alkenyl, (2-4C)alkynyl and trifluoroalkyl;
(B) each Y is independently selected from hydroxyl or a phenyl group that is optionally substituted with one or more groups selected from fluoro, (1-4C)alkyl and trifluoromethyl;
(C) each Y is independently selected from hydroxyl or a phenyl group that is optionally substituted with one or more groups selected from fluoro and trifluoromethyl.

5. The solid-phase support material of any preceding claim, wherein any one or more of the following statements (A) to (F) applies:
(A) Rₐ-Rₑ are independently hydrogen, hydroxyl, (1-4C)alkyl, 2-4C)alkenyl, (2-4C)alkynyl, fluoro or trifluoroalkyl;
(B) Rₐ-Rₑ are independently hydrogen, (1-4C)alkyl, fluoro or trifluoroalkyl;
(C) Rₐ-Rₑ are independently hydrogen, methyl, ethyl, tert-butyl, fluoro or trifluoromethyl;
(D) R_{f} is (1-4C)alkyl, trihaloalkyl or a phenyl group that is optionally substituted with one or more groups selected from hydroxyl, (1-4C)alkyl, halo and trihaloalkyl;
(E) R_{f} is (1-4C)alkyl, trihaloalkyl or p-toluenyl;
(F) R_{f} is methyl, ethyl, trifluoromethyl or p-toluenyl.

6. The solid-phase support material of any preceding claim, wherein either or both of the following statements (A) and (B) applies:
(A) the mole ratio of the compound of formula (I) or (II) to the solid polymethylaluminoxane within the solid-phase support material ranges from 0.0001:1 to 0.1:1;
(B) the mole ratio of the compound of formula (I) or (II) to the solid polymethylaluminoxane within the solid-phase support material ranges from 0.0001:1 to 0.05:1.

7. The solid-phase support material of any preceding claim, wherein either or both of the following statements (A) and (B) applies:
(A) the compound of formula (I) or (II) is selected from one or more of the following: 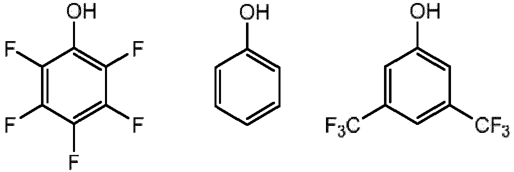 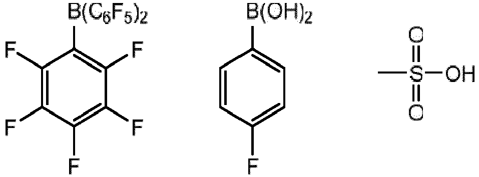
(B) the compound of formula (I) or (II) is selected from one or more of the following:

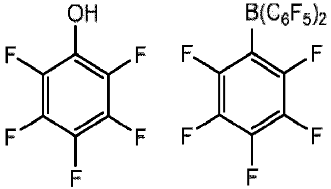

8. A method of preparing a solid-phase support material as claimed in any preceding claim, the method comprising the steps of:
a) providing a solid polymethylaluminoxane in a first solvent;
b) contacting the solid polymethylaluminoxane of step a) with one or more compounds of formula (I) or (II) as defined in any preceding claim; and
c) isolating the product formed from step b);
wherein the mole ratio of the compound of formula (I) or (II) to the solid polymethylaluminoxane used in step b) ranges from 0.0001:1 to 0.3:1.

9. The method of claim 8, wherein any one or more of the following statements (A) to (G) applies:
(A) the one or more compounds of formula (I) or (II) used in step b) is provided in a second solvent;
(B) step b) is conducted at a temperature of 18-150°C;
(C) step b) is conducted at a temperature of 18-50°C;
(D) the first solvent is selected from toluene, benzene and hexane;
(E) the first solvent is toluene;
(F) the second solvent is selected from toluene, benzene and hexane;
(G) the second solvent is toluene.

10. The method of claim 8 or 9, wherein step b) further comprises the step of sonicating the mixture of the solid polymethylaluminoxane and the one or more compounds of formula (I) or (II).

11. A use of the solid-phase support material as claimed in any of claims 1-7 as a solid-phase support for supporting a metallocene catalytic compound.

12. A catalytic composition comprising:
a) a compound of formula (III) shown below; and
b) a solid-phase support material as claimed in any of claims 1-7 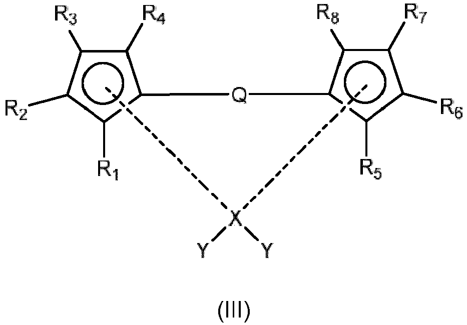 wherein
R₁ and R₂ are each independently hydrogen or (1-2C)alkyl;
R₃ and R₄ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl, (1-6C)alkoxy, aryl, heteroaryl, carbocyclic and heterocyclic, wherein each aryl, heteroaryl, carbocyclic and heterocyclic group is optionally substituted with one or more groups selected from (1-6C)alkyl, (2-6C)alkenyl, (2-6C)alkynyl, (1-6C)alkoxy, halo, amino, nitro, cyano, (1-6C)alkylamino, [(1-6C)alkyl]₂amino and -S(O)₂(1-6C)alkyl;
R₅ and R₆ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl, (1-6C)alkoxy, aryl, heteroaryl, carbocyclic and heterocyclic, wherein each aryl, heteroaryl, carbocyclic and heterocyclic group is optionally substituted with one or more groups selected from (1-6C)alkyl, (2-6C)alkenyl, (2-6C)alkynyl, (1-6C)alkoxy, halo, amino, nitro, cyano, (1-6C)alkylamino, [(1-6C)alkyl]₂amino and -S(O)₂(1-6C)alkyl;
R₇ and R₈ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl, (1-6C)alkoxy, aryl, heteroaryl, carbocyclic and heterocyclic, wherein each aryl, heteroaryl, carbocyclic and heterocyclic group is optionally substituted with one or more groups selected from (1-6C)alkyl, (2-6C)alkenyl, (2-6C)alkynyl, (1-6C)alkoxy, halo, amino, nitro, cyano, (1-6C)alkylamino, [(1-6C)alkyl]₂amino and -S(O)₂(1-6C)alkyl;
Q is absent, or is a bridging group selected from -CH₂- or -CH₂CH₂-, either or which may be optionally substituted with one or more groups selected from (1-4C)alkyl, (2-4C)alkenyl, (2-4C)alkynyl and aryl, or Q is a bridging group -Si(R₉)(R₁₀)-,
wherein Rg and R₁₀ are independently (1-4C)alkyl, (2-4C)alkenyl, (2-4C)alkynyl or aryl;
X is zirconium or hafnium; and
each Y group is independently selected from halo, hydride, (1-6C)alkyl, (1-6C)alkoxy, aryl or aryloxy, either or which is optionally substituted with one or more groups selected from (1-6C)alkyl and halo.

13. The catalytic composition of claim 12, wherein any one or more of the following statements (A) to (K) applies:
(A) R₁ and R₂ are each hydrogen;
(B) R₃ and R₄ are each independently hydrogen or (1-4C)alkyl, or R₃ and R₄ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl and (1-6C)alkoxy;
(C) R₃ and R₄ are each independently hydrogen or (1-4C)alkyl, or R₃ and R₄ are linked such that, when taken in combination with the atoms to which they are attached, they form a 6-membered fused aromatic ring optionally substituted with one or more groups selected from methyl, ethyl and tert-butyl;
(D) R₅ and R₆ are each independently hydrogen or (1-4C)alkyl, or R₅ and R₆ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl and (1-6C)alkoxy;
(E) R₅ and R₆ are each independently hydrogen or (1-4C)alkyl, or R₅ and R₆ are linked such that, when taken in combination with the atoms to which they are attached, they form a 6-membered fused aromatic ring optionally substituted with one or more groups selected from methyl, ethyl and tert-butyl;
(F) R₇ and R₈ are each independently hydrogen or (1-4C)alkyl, or R₇ and R₈ are linked such that, when taken in combination with the atoms to which they are attached, they form a 6-membered fused aromatic ring optionally substituted with one or more groups selected from (1-6C)alkyl, (2-6C)alkenyl, (2-6C)alkynyl and (1-6C)alkoxy;
(G) R₇ and R₈ are each independently hydrogen or (1-4C)alkyl, or R₇ and R₈ are linked such that, when taken in combination with the atoms to which they are attached, they form a 6-membered fused aromatic ring optionally substituted with one or more groups selected from methyl, ethyl and tert-butyl;
(H) Q is absent, or is a bridging group selected from -CH₂- or -CH₂CH₂-, either or which may be optionally substituted with one or more groups selected from (1-4C)alkyl and phenyl, or Q is a bridging group -Si(R₉)(R₁₀)-,
wherein Rg and R₁₀ are independently (1-4C)alkyl or aryl;
(I) X is zirconium;
(J) Y group is independently selected from halo;
(K) when Q is -CH₂CH₂-, R_{d} is selected from hydrogen, hydroxyl, (1-4C)alkyl, (2-4C)alkenyl, (2-4C)alkynyl and halo.

14. A use of a catalytic composition as claimed claim 12 or 13 as a polymerisation catalyst for the preparation of a polyolefin.

15. The use according to claim 14, wherein the polyolefin is polyethylene;
optionally wherein the polyolefin is a copolymer formed from ethene monomers comprising 1-10 wt%, by total weight of the monomer, of one or more (4-8C) α-olefin.

## Patentansprüche

1. Festphasen-Trägermaterial, das zum Tragen einer katalytischen Metallocenverbindung geeignet ist, wobei das Festphasen-Trägermaterial ein festes Polymethylaluminoxan umfasst, das durch Reaktion mit einer Verbindung der nachstehend gezeigten Formel (I) oder (II) modifiziert wurde: 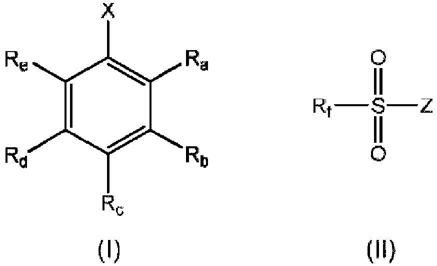 wobei
X Hydroxy oder eine Gruppe B(Y)₂ oder eine Gruppe Al(Y)₂ ist,
wobei jedes Y unabhängig aus Hydroxy, Phenyl und Naphthalinyl ausgewählt ist, von denen jedes gegebenenfalls mit einer oder mehreren Gruppen ausgewählt aus Halogen, Hydroxy, (1-4C)Alkyl, (2-4C)Alkenyl, (2-4C)Alkinyl und Trihalogenalkyl substituiert sein kann;
Rₐ und R_{b} unabhängig Wasserstoff, Hydroxy, (1-4C)Alkyl, (2-4C)Alkenyl, (2-4C)Alkinyl, Halogen oder Trihalogenalkyl sind, oder
Rₐ und R_{b} derart verknüpft sind, dass sie zusammen mit den Atomen, an die sie gebunden sind, einen 6-gliedrigen aromatischen Ring bilden, der gegebenenfalls durch eine oder mehrere Gruppen ausgewählt aus Halogen, (1-4C)Alkyl, (2-4C)Alkenyl, (2-4C)Alkinyl und Trihalogenalkyl substituiert ist;
R_{c}-Rₑ unabhängig Wasserstoff, Hydroxy, (1-4C)Alkyl, (2-4C)Alkenyl, (2-4C)Alkinyl, Halogen oder Trihalogenalkyl sind;
Z Hydroxy oder eine Gruppe -NRₓR_{y} ist,
wobei Rₓ und R_{y} unabhängig aus Wasserstoff und (1-4C)Alkyl ausgewählt sind;
R_{f} (1-4C)Alkyl, (2-4C)Alkenyl, (2-4C)Alkinyl, Trihalogenalkyl oder eine Phenylgruppe ist, die gegebenenfalls mit einer oder mehreren Gruppen ausgewählt aus Hydroxy, (1-4C)Alkyl, (2-4C)Alkenyl, (2-4C)Alkinyl, Halogen und Trihalogenalkyl substituiert ist;
wobei das Molverhältnis der Verbindung der Formel (I) oder (II) zu dem festen Polymethylaluminoxan innerhalb des Festphasen-Trägermaterials im Bereich von 0,0001 : 1 bis 0,3 : 1 liegt.

2. Festphasen-Trägermaterial nach Anspruch 1, wobei Z Hydroxy ist.

3. Festphasen-Trägermaterial nach Anspruch 1 oder 2, wobei, wenn X Hydroxy ist,
i) Rₐ-Rₑ jeweils unabhängig Halogen sind; oder
ii) Rₐ und R_{b} derart verknüpft sind, dass sie zusammen mit den Atomen, an die sie gebunden sind, einen 6-gliedrigen aromatischen Ring bilden, der gegebenenfalls durch eine oder mehrere Gruppen ausgewählt aus Halogen, (1-4C)Alkyl, (2-4C)Alkenyl, (2-4C)Alkinyl und Trihalogenalkyl substituiert ist und
R_{c}-Rₑ unabhängig Wasserstoff, Hydroxy, (1-4C)Alkyl, (2-4C)Alkenyl, (2-4C)Alkinyl, Halogen oder Trihalogenalkyl sind; oder
iii) Rₐ, R_{b} und Rₑ jeweils unabhängig Wasserstoff, Hydroxy, (1-4C)Alkyl, (2-4C)Alkenyl, (2-4C)Alkinyl, Halogen oder Trihalogenalkyl sind, und
R_{d} und Rₑ jeweils unabhängig Wasserstoff, Hydroxy, (1-4C)Alkyl, (2-4C)Alkenyl, (2-4C)Alkinyl oder Trihalogenalkyl sind; oder
iv) Rₐ, R_{b}, R_{d} und Rₑ jeweils unabhängig Wasserstoff, Hydroxy, (1-4C)Alkyl, (2-4C)Alkenyl, (2-4C)Alkinyl, Halogen oder Trihalogenalkyl sind, und
R_{c} Wasserstoff, Hydroxy, (1-4C)Alkyl, (2-4C)Alkenyl, (2-4C)Alkinyl oder Halogen ist.

4. Festphasen-Trägermaterial nach Anspruch 1, 2 oder 3, wobei eine oder mehrere der folgenden Aussagen (A) bis (C) zutreffen:
(A) jedes Y ist unabhängig aus Hydroxy oder einer Phenylgruppe, die gegebenenfalls mit einer oder mehreren Gruppen ausgewählt aus Fluor, Hydroxy, (1-4C)Alkyl, (2-4C)Alkenyl, (2-4C)Alkinyl und Trifluoralkyl substituiert ist, ausgewählt;
(B) jedes Y ist unabhängig aus Hydroxy oder einer Phenylgruppe, die gegebenenfalls mit einer oder mehreren Gruppen ausgewählt aus Fluor, (1-4C)-Alkyl und Trifluormethyl substituiert ist, ausgewählt;
(C) jedes Y ist unabhängig aus Hydroxy oder einer Phenylgruppe, die gegebenenfalls mit einer oder mehreren Gruppen ausgewählt aus Fluor und Trifluormethyl substituiert ist, ausgewählt.

5. Festphasen-Trägermaterial nach einem der vorhergehenden Ansprüche, wobei eine oder mehrere der folgenden Aussagen (A) bis (F) zutreffen:
(A) Rₐ-Rₑ sind unabhängig Wasserstoff, Hydroxy, (1-4C)Alkyl, (2-4C)Alkenyl, (2-4C)Alkinyl, Fluor oder Trifluoralkyl;
(B) Rₐ-Rₑ sind unabhängig Wasserstoff, (1-4C)Alkyl, Fluor oder Trifluoralkyl;
(C) Rₐ-Rₑ sind unabhängig Wasserstoff, Methyl, Ethyl, tert-Butyl, Fluor oder Trifluormethyl ;
(D) R_{f} ist (1-4C)Alkyl, Trihalogenalkyl oder eine Phenylgruppe, die gegebenenfalls mit einer oder mehreren Gruppen ausgewählt aus Hydroxy, (1-4C)Alkyl, Halogen und Trihalogenalkyl substituiert ist;
(E) R_{f} ist (1-4C)Alkyl, Trihalogenalkyl oder *p*-Toluenyl;
(F) R_{f} ist Methyl, Ethyl, Trifluormethyl oder *p*-Toluenyl.

6. Festphasen-Trägermaterial nach einem der vorhergehenden Ansprüche, wobei eine oder beide der folgenden Aussagen (A) und (B) zutreffen:
(A) das Molverhältnis der Verbindung der Formel (I) oder (II) zum festen Polymethylaluminoxan innerhalb des Festphasen-Trägermaterials liegt im Bereich von 0,0001 : 1 bis 0,1 : 1;
(B) das Molverhältnis der Verbindung der Formel (I) oder (II) zum festen Polymethylaluminoxan innerhalb des Festphasen-Trägermaterials liegt im Bereich von 0,0001 : 1 bis 0,05 : 1.

7. Festphasen-Trägermaterial nach einem der vorhergehenden Ansprüche, wobei eine oder beide der folgenden Aussagen (A) und (B) zutreffen:
(A) die Verbindung der Formel (I) oder (II) ist aus einem oder mehreren der Folgenden ausgewählt: 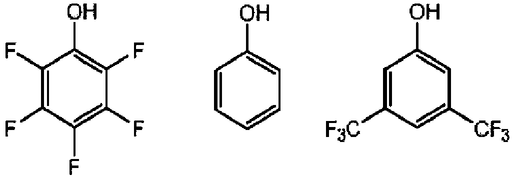 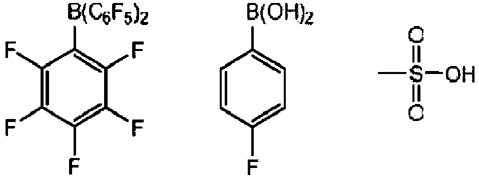
(B) die Verbindung der Formel (I) oder (II) ist aus einem oder mehreren der Folgenden ausgewählt:

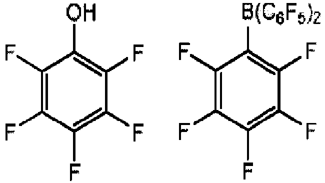

8. Verfahren zur Herstellung eines Festphasen-Trägermaterials nach einem der vorhergehenden Ansprüche, wobei das Verfahren die Schritte umfasst:
a) Bereitstellen eines festen Polymethylaluminoxans in einem ersten Lösungsmittel;
b) Inkontaktbringen des festen Polymethylaluminoxans von Schritt a) mit einer oder mehreren Verbindungen der Formel (I) oder (II), wie in einem der vorhergehenden Ansprüche definiert; und
c) Isolieren des in Schritt b) gebildeten Produkts;
wobei das Molverhältnis der Verbindung der Formel (I) oder (II) zu dem in Schritt b) verwendeten festen Polymethylaluminoxan im Bereich von 0,0001 : 1 bis 0,3 : 1 liegt.

9. Verfahren nach Anspruch 8, wobei eine oder mehrere der folgenden Aussagen (A) bis (G) zutreffen:
(A) die eine oder mehreren Verbindungen der Formel (I) oder (II), die in Schritt b) verwendet werden, werden in einem zweiten Lösungsmittel bereitgestellt;
(B) Schritt b) wird bei einer Temperatur von 18-150 °C durchgeführt;
(C) Schritt b) wird bei einer Temperatur von 18-50 °C durchgeführt;
(D) das erste Lösungsmittel ist ausgewählt aus Toluol, Benzol und Hexan;
(E) das erste Lösungsmittel ist Toluol;
(F) das zweite Lösungsmittel ist aus Toluol, Benzol und Hexan ausgewählt;
(G) das zweite Lösungsmittel ist Toluol.

10. Verfahren nach Anspruch 8 oder 9, wobei Schritt b) ferner den Schritt des Beschallens der Mischung des festen Polymethylaluminoxans und der einen oder mehreren Verbindungen der Formel (I) oder (II) umfasst.

11. Verwendung des Festphasen-Trägermaterials nach einem der Ansprüche 1-7 als Festphasenträger zum Tragen einer katalytischen Metallocenverbindung.

12. Katalytische Zusammensetzung, umfassend:
a) Verbindung der Formel (III), die nachfolgend gezeigt ist; und
b) ein Festphasen-Trägermaterial nach einem der Ansprüche 1-7 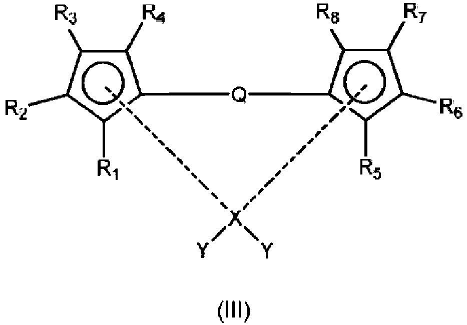 wobei
R₁ und R₂ jeweils unabhängig Wasserstoff oder (1-2C)Alkyl sind;
R₃ und R₄ jeweils unabhängig Wasserstoff oder (1-4C)Alkyl sind, oder R₃ und R₄ derart verknüpft sind, dass sie in Kombination mit den Atomen, an die sie gebunden sind, einen 6-gliedrigen kondensierten aromatischen Ring bilden, der gegebenenfalls mit einer oder mehreren Gruppen, die aus (1-6C)Alkyl, (2-6C)Alkenyl, (2-6C)Alkinyl, (1-6C)Alkoxy, Aryl, Heteroaryl, carbocyclisch und heterocyclisch ausgewählt sind, substituiert ist, wobei jede Aryl-, Heteroaryl-, carbocyclische und heterocyclische Gruppe gegebenenfalls mit einer oder mehreren Gruppen, die aus (1-6C)Alkyl, (2-6C)Alkenyl, (2-6C)Alkinyl, (1-6C)Alkoxy, Halogen, Amino, Nitro, Cyano, (1-6C)Alkylamino, [(1-6C)Alkyl]₂amino und -S(O)₂(1-6C)Alkyl ausgewählt sind, substituiert ist;
R₅ und R₆ jeweils unabhängig Wasserstoff oder (1-4C)Alkyl sind, oder R₃ und R₄ derart verknüpft sind, dass sie in Kombination mit den Atomen, an die sie gebunden sind, einen 6-gliedrigen kondensierten aromatischen Ring ausbilden, der gegebenenfalls mit einer oder mehreren Gruppen, die aus (1-6C)Alkyl, (2-6C)Alkenyl, (2-6C)Alkinyl, (1-6C)Alkoxy, Aryl, Heteroaryl, carbocyclisch und heterocyclisch ausgewählt sind, substituiert ist, wobei jede Aryl-, Heteroaryl-, carbocyclische und heterocyclische Gruppe gegebenenfalls mit einer oder mehreren Gruppen, die aus (1-6C)Alkyl, (2-6C)Alkenyl, (2-6C)Alkinyl, (1-6C)Alkoxy, Halogen, Amino, Nitro, Cyano, (1-6C)Alkylamino, [(1-6C)Alkyl]₂amino und -S(O)₂(1-6C)Alkyl ausgewählt sind, substituiert ist;
R₇ und R₈ jeweils unabhängig Wasserstoff oder (1-4C)Alkyl sind, oder R₃ und R₄ derart verknüpft sind, dass sie in Kombination mit den Atomen, an die sie gebunden sind, einen 6-gliedrigen kondensierten aromatischen Ring ausbilden, der gegebenenfalls mit einer oder mehreren Gruppen, die aus (1-6C)Alkyl, (2-6C)Alkenyl, (2-6C)Alkinyl, (1-6C)Alkoxy, Aryl, Heteroaryl, carbocyclisch und heterocyclisch ausgewählt sind, substituiert ist, wobei jede Aryl-, Heteroaryl-, carbocyclische und heterocyclische Gruppe gegebenenfalls mit einer oder mehreren Gruppen, die aus (1-6C)Alkyl, (2-6C)Alkenyl, (2-6C)Alkinyl, (1-6C)Alkoxy, Halogen, Amino, Nitro, Cyano, (1-6C)Alkylamino, [(1-6C)Alkyl]₂amino und -S(O)₂(1-6C)Alkyl ausgewählt sind, substituiert ist;
Q fehlt oder eine Brückengruppe ist, ausgewählt aus -CH₂- oder -CH₂CH₂-, wobei eine oder diese gegebenenfalls mit einer oder mehreren Gruppen ausgewählt aus (1-4C)Alkyl, (2-4C)Alkenyl, (2-4C)Alkinyl und Aryl substituiert sein können oder Q eine Brückengruppe -Si(R₉)(R₁₀)- ist,
wobei R₉ und R₁₀ unabhängig (1-4C)Alkyl, (2-4C)Alkenyl, (2-4C)Alkinyl oder Aryl sind;
X Zirkonium oder Hafnium ist; und
jede Y-Gruppe unabhängig aus Halogen, Hydrid, (1-6C)Alkyl, (1-6C)Alkoxy, Aryl oder Aryloxy ausgewählt ist, wobei eine davon oder alle gegebenenfalls mit einer oder mehreren Gruppen ausgewählt aus (1-6C)Alkyl und Halogen substituiert sind.

13. Katalytische Zusammensetzung nach Anspruch 12, wobei eine oder mehrere der folgenden Aussagen (A) bis (K) zutreffen:
(A) R₁ und R₂ sind jeweils Wasserstoff;
(B) R₃ und R₄ sind jeweils unabhängig Wasserstoff oder (1-4C)Alkyl, oder R₃ und R₄ sind derart verknüpft, dass sie in Kombination mit den Atomen, an die sie gebunden sind, einen 6-gliedrigen kondensierten aromatischen Ring bilden, der gegebenenfalls mit einer oder mehreren Gruppen ausgewählt aus (1-6C)Alkyl, (2-6C)Alkenyl, (2-6C)Alkinyl und (1-6C)Alkoxy substituiert ist;
(C) R₃ und R₄ sind jeweils unabhängig Wasserstoff oder (1-4C)Alkyl, oder R₃ und R₄ sind derart verknüpft, dass sie in Kombination mit den Atomen, an die sie gebunden sind, einen 6-gliedrigen kondensierten aromatischen Ring bilden, der gegebenenfalls mit einer oder mehreren Gruppen ausgewählt aus Methyl, Ethyl und tert-Butyl substituiert ist;
(D) R₅ und R₆ sind jeweils unabhängig Wasserstoff oder (1-4C)Alkyl, oder R₅ und R₆ sind derart verknüpft, dass sie in Kombination mit den Atomen, an die sie gebunden sind, einen 6-gliedrigen kondensierten aromatischen Ring bilden, der gegebenenfalls mit einer oder mehreren Gruppen ausgewählt aus (1-6C)Alkyl, (2-6C)Alkenyl, (2-6C)Alkinyl und (1-6C)Alkoxy substituiert ist;
(E) R₅ und R₆ sind jeweils unabhängig Wasserstoff oder (1-4C)Alkyl, oder R₅ und R₆ sind derart verknüpft, dass sie in Kombination mit den Atomen, an die sie gebunden sind, einen 6-gliedrigen kondensierten aromatischen Ring bilden, der gegebenenfalls mit einer oder mehreren Gruppen ausgewählt aus Methyl, Ethyl und tert-Butyl substituiert ist;
(F) R₇ und R₈ sind jeweils unabhängig Wasserstoff oder (1-4C)Alkyl, oder R₇ und R₈ sind derart verknüpft, dass sie in Kombination mit den Atomen, an die sie gebunden sind, einen 6-gliedrigen kondensierten aromatischen Ring bilden, der gegebenenfalls mit einer oder mehreren Gruppen ausgewählt aus (1-6C)Alkyl, (2-6C)Alkenyl, (2-6C)Alkinyl und (1-6C)Alkoxy substituiert ist;
(G) R₇ und R₈ sind jeweils unabhängig Wasserstoff oder (1-4C)Alkyl, oder R₇ und R₈ sind derart verknüpft, dass sie in Kombination mit den Atomen, an die sie gebunden sind, einen 6-gliedrigen kondensierten aromatischen Ring bilden, der gegebenenfalls mit einer oder mehreren Gruppen ausgewählt aus Methyl, Ethyl und tert-Butyl substituiert ist;
(H) Q fehlt oder ist eine Brückengruppe ausgewählt aus -CH₂- oder -CH₂CH₂-, wobei eine davon oder beide gegebenenfalls mit einer oder mehreren Gruppen ausgewählt aus (1-4C)Alkyl und Phenyl substituiert sein können, oder Q ist eine Brückengruppe -Si (R₉)(R₁₀)-,
wobei R₉ und R₁₀ unabhängig (1-4C)Alkyl oder Aryl sind;
(I) X ist Zirkonium;
(J) die Y-Gruppe ist unabhängig aus Halogen ausgewählt:
(K) wenn Q -CH₂CH₂- ist, ist R_{d} ausgewählt aus Wasserstoff, Hydroxy, (1-4C)Alkyl, (2-4C)Alkenyl, (2-4C)Alkinyl und Halogen.

14. Verwendung einer katalytischen Zusammensetzung nach Anspruch 12 oder 13 als Polymerisationskatalysator zur Herstellung eines Polyolefins.

15. Verwendung nach Anspruch 14, wobei das Polyolefin Polyethylen ist;
wobei das Polyolefin gegebenenfalls ein Copolymer ist, das aus Ethenmonomeren gebildet ist, die 1-10 Gew.-%, bezogen auf das Gesamtgewicht des Monomers, eines oder mehrerer (4-8C)-α-Olefine umfassen.

## Revendications

1. Matériau support en phase solide destiné à supporter un composé catalytique de métallocène, le matériau support en phase solide comprenant un polyméthylaluminoxane solide modifié par une réaction avec un composé de formule (I) ou (II) présenté ci-dessous : 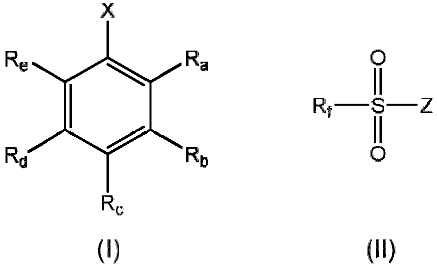 dans lesquelles
X représente un groupe hydroxy ou un groupe B(Y)₂ ou un groupe Al(Y)₂,
dans lesquels chaque Y est indépendamment choisi parmi les groupes hydroxy, phényle et naphtalényle, l'un quelconque desquels peut être substitué par un ou plusieurs groupes choisis parmi les groupes halogéno, hydroxy, (1 à 4C)alkyle, (2 à 4C)alcényle, (2 à 4C)alcynyle et trihalogénoalkyle ;
Rₐ et R_{b} représentent indépendamment un atome d'hydrogène, un groupe hydroxy, (1 à 4C)alkyle, (2 à 4C)alcényle, (2 à 4C)alcynyle, halogéno ou trihalogénoalkyle, ou
Rₐ et R_{b} sont liés, de sorte que, lorsqu'ils sont pris avec les atomes auxquels ils sont liés, ils forment un noyau aromatique à 6 chaînons qui est éventuellement substitué par un ou plusieurs groupes choisis parmi les groupes halogéno, (1 à 4C)alkyle, (2 à 4C)alcényle, (2 à 4C)alcynyle et trihalogénoalkyle ;
R_{c} à Rₑ représentent chacun indépendamment un atome d'hydrogène, un groupe hydroxy, (1 à 4C)alkyle, (2 à 4C)alcényle, (2 à 4C)alcynyle, halogéno ou trihalogénoalkyle ;
Z représente un groupe hydroxy ou un groupe -NRₓR_{y},
dans lequel Rₓ et R_{y} sont indépendamment choisis parmi un atome d'hydrogène et un groupe (1 à 4C)alkyle ;
R_{f} représente un groupe (1 à 4C)alkyle, (2 à 4C)alcényle, (2 à 4C)alcynyle, trihalogénoalkyle ou un groupe phényle qui est éventuellement substitué par un ou plusieurs groupes choisis parmi un groupe hydroxy, (1 à 4C)alkyle, (2 à 4C)alcényle, (2 à 4C)alcynyle, halogéno et trihalogénoalkyle ;
le rapport molaire du composé de formule (I) ou (II) sur le polyméthylaluminoxane solide dans le matériau support en phase solide allant de 0,0001:1 à 0,3:1.

2. Matériau support en phase solide selon la revendication 1, Z représentant un groupe hydroxy.

3. Matériau support en phase solide selon la revendication 1 ou 2, lorsque X représente un groupe hydroxy
i) Rₐ à Rₑ représentant chacun indépendamment un groupe halogéno ; ou
ii) Rₐ et R_{b} étant liés, de sorte que, lorsqu'ils sont pris avec les atomes auxquels ils sont liés, ils forment un noyau aromatique à 6 chaînons qui est éventuellement substitué par un ou plusieurs groupes choisis parmi les groupes halogéno, (1 à 4C)alkyle, (2 à 4C)alcényle, (2 à 4C)alcynyle et trihalogénoalkyle, et
R_{c} à Rₑ représentant indépendamment un atome d'hydrogène, un groupe hydroxy, (1 à 4C)alkyle, (2 à 4C)alcényle, (2 à 4C)alcynyle, halogéno ou trihalogénoalkyle ; ou
iii) Rₐ, R_{b} et Rₑ représentant chacun indépendamment un atome d'hydrogène, un groupe hydroxy, (1 à 4C)alkyle, (2 à 4C)alcényle, (2 à 4C)alcynyle, halogéno ou trihalogénoalkyle, et
R_{d} et Rₑ représentant chacun indépendamment un atome d'hydrogène, un groupe hydroxy, (1 à 4C)alkyle, (2 à 4C)alcényle, (2 à 4C)alcynyle ou trihalogénoalkyle ; ou
iv) Rₐ, R_{b}, R_{d} et Rₑ représentant chacun indépendamment un atome d'hydrogène, un groupe hydroxy, (1 à 4C)alkyle, (2 à 4C)alcényle, (2 à 4C)alcynyle, halogéno ou trihalogénoalkyle, et
R_{c} représentant un atome d'hydrogène, un groupe hydroxy, (1 à 4C)alkyle, (2 à 4C)alcényle, (2 à 4C)alcynyle ou halogéno.

4. Matériau support en phase solide selon la revendication 1, 2 ou 3, l'une quelconque ou plusieurs des affirmations (A) à (C) suivantes s'appliquant :
(A) chaque Y est indépendamment choisi parmi un groupe hydroxy ou un groupe phényle qui est éventuellement substitué par un ou plusieurs groupes choisis parmi les groupes fluoro, hydroxy, (1 à 4C)alkyle, (2 à 4C)alcényle, (2 à 4C)alcynyle et trifluoroalkyle ;
(B) chaque Y est indépendamment choisi parmi un groupe hydroxy ou un groupe phényle qui est éventuellement substitué par un ou plusieurs groupes choisis parmi les groupes fluoro, (1 à 4C)alkyle et trifluorométhyle ;
(C) chaque Y est indépendamment choisi parmi un groupe hydroxy ou un groupe phényle qui est éventuellement substitué par un ou plusieurs groupes choisis parmi les groupes fluoro et trifluorométhyle.

5. Matériau support en phase solide selon l'une quelconque des revendications précédentes, l'une quelconque ou plusieurs des affirmations (A) à (F) suivantes s'appliquant :
(A) Rₐ à Rₑ représentent indépendamment un atome d'hydrogène, un groupe hydroxy, (1 à 4C)alkyle, (2 à 4C)alcényle, (2 à 4C)alcynyle, fluoro ou trifluoroalkyle ;
(B) Rₐ à Rₑ représentent indépendamment un atome d'hydrogène, un groupe (1 à 4C)alkyle, fluoro ou trifluoroalkyle ;
(C) Rₐ à Rₑ représentent indépendamment un atome d'hydrogène, un groupe méthyle, éthyle, tert-butyle, fluoro ou trifluorométhyle ;
(D) R_{f} représente un groupe (1 à 4C)alkyle, trihalogénoalkyle ou un groupe phényle qui est éventuellement substitué par un ou plusieurs groupes choisis parmi les groupes hydroxy, (1 à 4C)alkyle, halogéno et trihalogénoalkyle ;
(E) R_{f} représente un groupe (1 à 4C)alkyle, trihalogénoalkyle ou *p*-toluényle ;
(F) R_{f} représente un groupe méthyle, éthyle, trifluorométhyle ou *p*-toluényle.

6. Matériau support en phase solide selon l'une quelconque des revendications précédentes, l'une et/ou l'autre parmi les affirmations (A) à (B) suivantes s'appliquant :
(A) le rapport molaire du composé de formule (I) ou (II) sur le polyméthylaluminoxane solide dans le matériau support en phase solide va de 0,0001:1 à 0,1:1 ;
(B) le rapport molaire du composé de formule (I) ou (II) sur le polyméthylaluminoxane solide dans le matériau support en phase solide va de 0,0001:1 à 0,05:1.

7. Matériau support en phase solide selon l'une quelconque des revendications précédentes, l'une et/ou l'autre parmi les affirmations (A) à (B) suivantes s'appliquant :
(A) le composé de formule (I) ou (II) est choisi parmi l'un ou plusieurs des composés suivants : 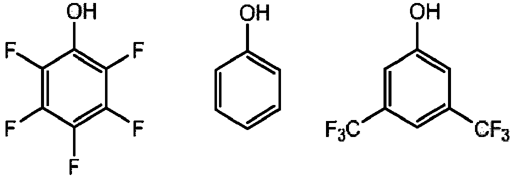 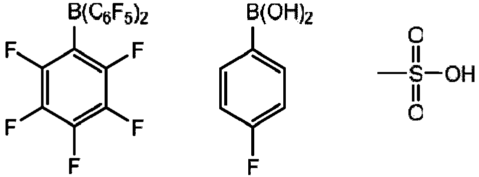
(B) le composé de formule (I) ou (II) est choisi parmi l'un ou plusieurs des composés suivants :

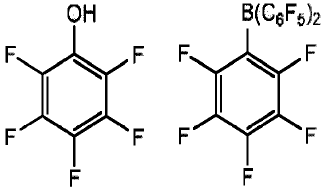

8. Procédé de préparation d'un matériau support en phase solide selon l'une quelconque des revendications précédentes, le procédé comprenant les étapes de :
a) fourniture d'un polyméthylaluminoxane solide dans un premier solvant ;
b) mise en contact du polyméthylaluminoxane solide de l'étape a) avec un ou plusieurs composés de formule (I) ou (II) définis dans l'une quelconque des revendications précédentes ; et
c) isolement du produit formé à partir de l'étape b) ;
ledit rapport molaire du composé de formule (I) ou (II) sur le polyméthylaluminoxane solide utilisé à l'étape b) allant de 0,0001:1 à 0,3:1.

9. Procédé selon la revendication 8, l'une quelconque ou plusieurs des affirmations (A) à (G) suivantes s'appliquant :
(A) lesdits un ou plusieurs composés de formule (I) ou (II) utilisés à l'étape b) sont fournis dans un second solvant ;
(B) l'étape b) est réalisée à une température de 18 à 150°C ;
(C) l'étape b) est réalisée à une température de 18 à 50°C ;
(D) le premier solvant est choisi parmi le toluène, le benzène et l'hexane ;
(E) le premier solvant est le toluène ;
(F) le second solvant est choisi parmi le toluène, le benzène et l'hexane ;
(G) le second solvant est le toluène.

10. Procédé selon la revendication 8 ou 9, ladite étape b) comprenant en outre l'étape de sonification du mélange du polyméthylaluminoxane solide et desdits un ou plusieurs composés de formule (I) ou (II).

11. Utilisation du matériau support en phase solide selon l'une quelconque des revendications 1 à 7 en tant que support en phase solide pour supporter un composé catalytique de métallocène.

12. Composition catalytique comprenant :
a) un composé de formule (III) présenté ci-dessous ; et
b) un matériau support en phase solide selon l'une quelconque des revendications 1 à 7 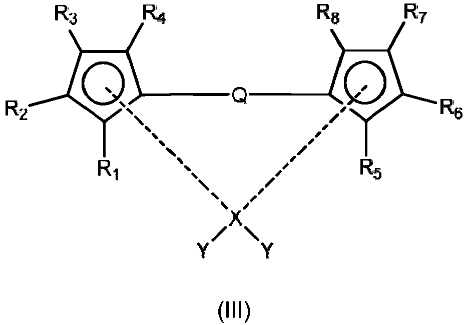 dans laquelle
R₁ et R₂ représentent chacun indépendamment un atome d'hydrogène ou un groupe (1 à 2C)alkyle ;
R₃ et R₄ représentent chacun indépendamment un atome d'hydrogène ou un groupe (1 à 4C)alkyle, ou R₃ et R₄ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes (1 à 6C)alkyle, (2 à 6C)alcényle, (2 à 6C)alcynyle, (1 à 6C)alcoxy, aryle, hétéroaryle, carbocyclique et hétérocyclique, chaque groupe aryle, hétéroaryle, carbocyclique et hétérocyclique étant éventuellement substitué par un ou plusieurs groupes choisis parmi les groupes (1 à 6C)alkyle, (2 à 6C)alcényle, (2 à 6C)alcynyle, (1 à 6C)alcoxy, halogéno, amino, nitro, cyano, (1 à 6C)alkylamino, [(1 à 6C)alkyl]₂amino et-S(O)₂(1 à 6C)alkyle ;
R₅ et R₆ représentent chacun indépendamment un atome d'hydrogène ou un groupe (1 à 4C)alkyle, ou R₃ et R₄ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes (1 à 6C)alkyle, (2 à 6C)alcényle, (2 à 6C)alcynyle, (1 à 6C)alcoxy, aryle, hétéroaryle, carbocyclique et hétérocyclique, chaque groupe aryle, hétéroaryle, carbocyclique et hétérocyclique étant éventuellement substitué par un ou plusieurs groupes choisis parmi les groupes (1 à 6C)alkyle, (2 à 6C)alcényle, (2 à 6C)alcynyle, (1 à 6C)alcoxy, halogéno, amino, nitro, cyano, (1 à 6C)alkylamino, [(1 à 6C)alkyl]₂amino et-S(O)₂(1 à 6C)alkyle ;
R₇ et R₈ représentent chacun indépendamment un atome d'hydrogène ou un groupe (1 à 4C)alkyle, ou R₃ et R₄ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes (1 à 6C)alkyle, (2 à 6C)alcényle, (2 à 6C)alcynyle, (1 à 6C)alcoxy, aryle, hétéroaryle, carbocyclique et hétérocyclique, chaque groupe aryle, hétéroaryle, carbocyclique et hétérocyclique étant éventuellement substitué par un ou plusieurs groupes choisis parmi les groupes (1 à 6C)alkyle, (2 à 6C)alcényle, (2 à 6C)alcynyle, (1 à 6C)alcoxy, halogéno, amino, nitro, cyano, (1 à 6C)alkylamino, [(1 à 6C)alkyl]₂amino et-S(O)₂(1 à 6C)alkyle ;
Q est absent, ou représente un groupe de pontage choisi parmi un groupe -CH₂- ou-CH₂CH₂-, l'un ou l'autre ou lequel peut être éventuellement substitué par un ou plusieurs groupes choisis parmi les groupes (1 à 4C)alkyle, (2 à 4C)alcényle, (2 à 4C)alcynyle et aryle, ou Q représente un groupe de pontage -Si(R₉)(R₁₀)-,
R₉ et R₁₀ représentant indépendamment un groupe (1 à 4C)alkyle, (2 à 4C)alcényle, (2 à 4C)alcynyle ou aryle ;
X représente un atome de zirconium ou de hafnium ; et
chaque Y est indépendamment choisi parmi un groupe halogéno, hydrure, (1 à 6C)alkyle, (1 à 6C)alcoxy, aryle ou aryloxy, l'un ou l'autre ou lequel est éventuellement substitué par un ou plusieurs groupes choisis parmi les groupes (1 à 6C)alkyle et halogéno.

13. Composition catalytique selon la revendication 12, l'une quelconque ou plusieurs des affirmations (A) à (K) suivantes s'appliquant :
(A) R₁ et R₂ représentent chacun un atome d'hydrogène ;
(B) R₃ et R₄ représentent chacun indépendamment un atome d'hydrogène ou un groupe (1 à 4C)alkyle, ou R₃ et R₄ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes (1 à 6C)alkyle, (2 à 6C)alcényle, (2 à 6C)alcynyle et (1 à 6C)alcoxy ;
(C) R₃ et R₄ représentent chacun indépendamment un atome d'hydrogène ou un groupe (1 à 4C)alkyle, ou R₃ et R₄ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes méthyle, éthyle et tert-butyle ;
(D) R₅ et R₆ représentent chacun indépendamment un atome d'hydrogène ou un groupe (1 à 4C)alkyle, ou R₅ et R₅ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes (1 à 6C)alkyle, (2 à 6C)alcényle, (2 à 6C)alcynyle et (1 à 6C)alcoxy ;
(E) R₅ et R₆ représentent chacun indépendamment un atome d'hydrogène ou un groupe (1 à 4C)alkyle, ou R₅ et R₆ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes méthyle, éthyle et tert-butyle ;
(F) R₇ et R₈ représentent chacun indépendamment un atome d'hydrogène ou un groupe (1 à 4C)alkyle, ou R₇ et R₈ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes (1 à 6C)alkyle, (2 à 6C)alcényle, (2 à 6C)alcynyle et (1 à 6C)alcoxy ;
(G) R₇ et R₈ représentent chacun indépendamment un atome d'hydrogène ou un groupe (1 à 4C)alkyle, ou R₇ et R₈ sont liés de sorte que, lorsqu'ils sont pris en combinaison avec les atomes auxquels ils sont liés, ils forment un noyau aromatique fusionné à 6 chaînons éventuellement substitué par un ou plusieurs groupes choisis parmi les groupes méthyle, éthyle et tert-butyle ;
(H) Q est absent, ou représente un groupe de pontage choisi parmi un groupe -CH₂- ou -CH₂CH₂-, l'un ou l'autre ou lequel peut être éventuellement substitué par un ou plusieurs groupes choisis parmi les groupes (1 à 4C)alkyle et phényle, ou Q représente un groupe de pontage - Si(R₉)(R₁₀)-,
R₉ et R₁₀ représentant indépendamment un groupe (1 à 4C)alkyle ou aryle ;
(I) X représente un atome de zirconium ;
(J) Y est indépendamment choisi parmi un groupe halogéno ;
(K) lorsque Q représente un groupe -CH₂CH₂-, R_{d} est choisi parmi un atome d'hydrogène, un groupe hydroxy, (1 à 4C)alkyle, (2 à 4C)alcényle, (2 à 4C)alcynyle et halogéno.

14. Utilisation d'une composition catalytique selon la revendication 12 ou 13 comme catalyseur de polymérisation pour la préparation d'une polyoléfine.

15. Utilisation selon la revendication 14, ladite polyoléfine étant le polyéthylène ; éventuellement ladite polyoléfine étant un copolymère formé de monomères éthène comprenant 1 à 10 % en poids, par poids total du monomère, d'une ou plusieurs (4 à 8C) α-oléfines.
